# EUROPEAN PATENT SPECIFICATION

(11) **EP 4 203 982 B1**
(45) Date of publication and mention of the grant of the patent: **12.02.2025**
(21) Application number: 21772984.7
(22) Date of filing: 27.08.2021
(51) Int. Cl.: A61K 35/20, A61K 38/01, A61K 38/17, A61K 38/39, A61P 19/00, A61P 21/00, A61P 29/00

(54) **COMBINATION OF COLLAGEN PEPTIDE AND WHEY PROTEIN FOR USE IN A CONDITION OF THE SKELETAL TISSUE**
KOMBINATION AUS KOLLAGENPEPTID UND MOLKEPROTEIN ZUR VERWENDUNG IN SKELETTGEWEBE
COMBINAISON DE PEPTIDE DE COLLAGÈNE ET DE PROTÉINE DE LACTOSÉRUM DESTINÉE À ÊTRE UTILISÉE DANS UNE AFFECTION DU TISSU SQUELETTIQUE

(30) Priority: 27.08.2020 JP 2020143653
(43) Date of publication of application: 05.07.2023
(73) Proprietor: Rousselot B.V., 5691 NM Son (NL)
(72) Inventor: NOGUCHI, Miwa, TOKYO, 103-0001 (JP); ASANO, Momoko, TOKYO, 103-0001 (JP); SUGIMOTO, Tetsuya, TOKYO, 103-0001 (JP); OSA, Shukichi, TOKYO, 103-0001 (JP); PRAWITT, Janne, 9000 GENT (BE)
(74) Representative: EP&C
(86) International application number: PCT/EP2021/073700
(87) International publication number: WO 2022/043476

(56) References cited:
- EP-A1- 3 666 298
- US-A1- 2009 298 767
- CLIFFORD TOM ET AL: "The effects of collagen peptides on muscle damage, inflammation and bone turnover following exercise: a randomized, controlled trial", AMINO ACIDS, SPRINGER VERLAG, AU, vol. 51, no. 4, 19 February 2019 (2019-02-19), pages 691 - 704, XP036750622, ISSN: 0939-4451, [retrieved on 20190219], DOI: 10.1007/S00726-019-02706-5
- HOSSAM EBAID ET AL: "Whey protein enhances normal inflammatory responses during cutaneous wound healing in diabetic rats", LIPIDS IN HEALTH AND DISEASE, BIOMED CENTRAL, LONDON, GB, vol. 10, no. 1, 14 December 2011 (2011-12-14), pages 235, XP021093754, ISSN: 1476-511X, DOI: 10.1186/1476-511X-10-235
- HARTOG ANITA ET AL: "Collagen hydrolysate inhibits zymosan-induced inflammation", EXPERIMENTAL BIOLOGY AND MEDICINE, vol. 238, no. 7, 20 July 2013 (2013-07-20), GB, pages 798 - 802, XP055920740, ISSN: 1535-3702, Retrieved from the Internet <URL:http://journals.sagepub.com/doi/full-xml/10.1177/1535370213480740> DOI: 10.1177/1535370213480740
- KERASIOTI EFTHALIA ET AL: "Anti-inflammatory effects of a special carbohydrate-whey protein cake after exhaustive cycling in hu", FOOD AND CHEMICAL TOXICOLOGY, PERGAMON, GB, vol. 61, 27 January 2013 (2013-01-27), pages 42 - 46, XP028789443, ISSN: 0278-6915, DOI: 10.1016/J.FCT.2013.01.023
- CASTRO G.A. ET AL: "Anti-Ulcerogenic Effect of a Whey Protein Isolate and Collagen Hydrolysates Against Ethanol Ulcerative Lesions on Oral Administration to Rats", JOURNAL OF MEDICINAL FOOD, vol. 13, no. 1, 5 February 2010 (2010-02-05), US, pages 83 - 90, XP055858792, ISSN: 1096-620X, Retrieved from the Internet <URL:https://www.liebertpub.com/doi/pdfplus/10.1089/jmf.2008.0277> DOI: 10.1089/jmf.2008.0277
- BONGIOVANNI TINDARO ET AL: "Nutritional interventions for reducing the signs and symptoms of exercise-induced muscle damage and accelerate recovery in athletes: current knowledge, practical application and future perspectives", EUROPEAN JOURNAL OF APPLIED PHYSIOLOGY, SPRINGER VERLAG, HEIDELBERG, DE, vol. 120, no. 9, 13 July 2020 (2020-07-13), pages 1965 - 1996, XP037216079, ISSN: 1439-6319, [retrieved on 20200713], DOI: 10.1007/S00421-020-04432-3
- SHAW GREGORY ET AL: "Vitamin C-enriched gelatin supplementation before intermittent activity augments collagen synthesis", THE AMERICAN JOURNAL OF CLINICAL NUTRITION, VOLUME 105, ISSUE 1, JANUARY 2017, PAGES 136-143, 16 November 2016 (2016-11-16), XP055859440, Retrieved from the Internet <URL:https://watermark.silverchair.com/ajcn138594.pdf?token=AQECAHi208BE49Ooan9kkhW_Ercy7Dm3ZL_9Cf3qfKAc485ysgAAAuMwggLfBgkqhkiG9w0BBwagggLQMIICzAIBADCCAsUGCSqGSIb3DQEHATAeBglghkgBZQMEAS4wEQQMHJi1Ll4G3KebnibaAgEQgIIClo6PLaE1qq1M2RoVvjgxRgfaywy5hIXN7cW7IcqTC7JeKY9k0vInR3AaF2vPfPmYy8tGB4aEf183ZGzJSXZMtnZ8b> [retrieved on 20211109], DOI: https://doi.org/10.3945/ajcn.116.138594
- BUCKLEY J D ET AL: "Supplementation with a whey protein hydrolysate enhances recovery of muscle force-generating capacity following eccentric exercise", JOURNAL OF SCIENCE AND MEDICINE IN SPORT, ELSEVIER, AMSTERDAM, NL, vol. 13, no. 1, 1 January 2010 (2010-01-01), pages 178 - 181, XP026813282, ISSN: 1440-2440, [retrieved on 20080902]
- TASAKA TORU ET AL: "Concentration-dependent Activation of Inflammatory/Anti-inflammatory Functions of Macrophages by Hydrolyzed Whey Protein", ANTICANCER RESEARCH, vol. 38, no. 7, 1 July 2018 (2018-07-01), GR, pages 4299 - 4304, XP055859494, ISSN: 0250-7005, Retrieved from the Internet <URL:https://ar.iiarjournals.org/content/anticanres/38/7/4299.full.pdf> DOI: 10.21873/anticanres.12728

## Description

### Technical field

The present disclosure relates to a combination for alleviating, preventing, or treating tissue inflammation, including the conditions and the associated symptoms associated therewith or resulting thereof. The present disclosure relates in the first place to tissue inflammation and related conditions developing in skeletal muscles and connective tissues, resulting from exercise or the like.

### Background

The skeletal tissues of the body comprise the skeletal muscle, bone, ligamentous, fibrous (e.g. tendons), and cartilaginous tissue. Its major functions are body support, facilitation of movement, protection of internal organs, storage of minerals and fat, and blood cell formation. Considering the many functions of the skeletal system and its components, its disease may have a drastic impact on one's quality of life.

Skeletal tissue diseases may be associated with abnormal tissue remodelling and/or tissue breakdown. A number of studies have for example reported excessive increases in muscle and tendon collagen turnover markers following muscle-damaging exercise, indicative of extensive degradation and remodelling in the extracellular matrix (ECM). The ECM provides the structural component of skeletal tissues, wherein collagen is typically the most abundant. For example, type I collagen is a predominant protein found in muscle, bone, and tendon, whereas type II collagen is a predominant protein found in the ECM of cartilage. Typically, tissue atrophy and disease occurs when there is a disturbance in the activity in anabolic and catabolic pathways within the ECM, more specifically, when the rate of ECM protein degradation exceeds the rate of ECM protein synthesis. This seems caused by a disruption of the balance between collagen synthesis and the activity of matrix metalloproteinases (MMPs) and tissue inhibitors of metalloproteinases (TIMPs) may result in chronic injuries, which currently have limited treatment options (Miller et al. J Physiol 567(3):1021-1033).

Skeletal tissue disease has particular large consequences in subjects undergoing strenuous physical activities, wherein it may not only reduce athletic performance, but also affect daily activities. For example, skeletal muscle damage may be caused by muscle rupture or muscle strain (of which there are various types), and is said to occur widely in various age groups and sports. In particular, athletes are often forced to withdraw from competition for a long period of time, so it is an important issue to prevent muscle damage and promote early recovery from this muscle damage. Microscopic damage (microdamage) of muscles has been considered as one of the causes of internal muscle damage in recent years. This mainly refers to the phenomenon in which the fibers that make up the muscle (muscle fibers) and the surrounding connective tissue are finely damaged, and it is considered that the accumulation of microdamage in the muscle tends to cause muscle strain.

Some methods have been reported that specifically aim at promoting the recovery of damaged skeletal tissue, including damaged skeletal muscle tissue. For example, there is a report of using botulinum toxin, which is a neurotoxin (patent document 1: no. WO0228425A2). By injecting Clostridium botulinum toxin into the muscle, it temporarily paralyzes and immobilizes the muscle, thereby accelerating recovery. In addition, the use of a novel amino-substituted benzonitrile derivative that bind to an androgen receptor (patent document 2: no. WO2005108351A1) has been reported, but it is a medicine mainly effective in the treatment of fractures or osteoporotic fractures, and muscle damage has been shown as an incidental effect. Further, the use of N-(3,4-dimethoxycinnamoyl) anthranilic acid (generic name: tranilast) known as a medicine indicated for bronchial asthma and the like (patent document 3: publication no. WO2004017953A1) has been reported, but it is a medicine that has been shown to have the effect of adjusting the balance between myocyte proliferation and myofibroblast proliferation by suppressing myofibroblastation in the damaged area and restoring the original muscle tissue. Incidentally, as a food material, the use of an amino acid mixed composition containing BCAA (branched chain amino acid) (patent document 4: no. WO2013021891A1 has been reported.

Castro et al. (J Med Food. 2010 Feb;13(1):83-90) discloses a use of whey protein isolate and collagen hydrolysates for treating ethanol ulcerative lesions in rats. Bongiovanni et al. (Eur J Appl Physiol. 2020 Sep;120(9):1965-1996) discloses use of whey protein or collagen-specific peptides as interventions in ameliorating exercise-induced muscle damage (EIMD). US 2009/298767A1 discloses a formulation comprising whey protein for use in treating muscle damage or for enhancing recovery following muscle damage. Shaw et al. (Am J Clin Nutr. 2017 Jan;105(1):136-143) discloses use of vitamin C in combination with gelatin for enhancing collagen synthesis after exercise. Buckley et al. (J Sci Med Sport. 2010 Jan;13(1):178-81.) discloses a use of a hydrolysate of whey protein for improving recovery and tissue repair after heavy exercise. EP3666298A1 discloses a collagen formulation for the treatment of musculoskeletal pathologies, including tendinitis, synovitis and (osteo)arthritis. Hossam Ebaid et al. (Lipids Health Dis. 2011 Dec 14:10:235) discloses that whey protein enhances the normal inflammatory response during wound healing in diabetic mice, restoring the levels of oxidative stress and inflammatory cytokines. Hartog et al. (Exp Biol Med -Maywood. 2013 Jul;238(7):798-802) discloses that collagen peptide modulates the inflammatory responses, which may be constituted by inhibiting pro-inflammatory cytokine production mediated by GlyR. Clifford et al. (Amino Acids. 2019 Apr;51(4):691-704) discloses the clinical effects of collagen peptide on EIMD. Kerasioti et al. (Food Chem Toxicol. 2013 Nov:61:42-6) discloses the effect of a cake consisting of carbohydrate and whey protein on inflammatory markers after exhaustive cycling.

There are several limitations to the methods proposed to promote recovery of damaged skeletal tissue. First, it is not realistic to use the botulinum toxin described in Patent Document 1 or the pharmaceutical compounds described in Patent Documents 2 and 3 on a daily basis. Further, the amino acid of Patent Document 4 has a problem of bitterness when ingested, and in particular, BCAA has a peculiar bitterness and astringent taste, which is a problem from the viewpoint of consumer preference.

Currently, there is a lack of an effective method to alleviate, prevent or treat skeletal diseases or conditions, in particular skeletal muscle diseases or conditions, in particular those which may not heal by simple rest (e.g. necessitating a therapeutic method).

### Summary

The invention is set out as in the appended claims.

Whey protein is a supplement rich in amino acids. It is commonly ingested before or after exercise, as it is believed to prevent muscle degeneration and promote protein synthesis. Using a mouse model of cardiotoxin-induced tissue inflammation, the current inventors found that whey protein, contrary to common belief, may have undesirable pro-inflammatory effects which have been largely overlooked to date.

At the same time, the current inventors found surprisingly that the combination of collagen peptide and whey protein reverses these pro-inflammatory effects of whey protein. The present inventors have found that, the combined administration of collagen peptide and whey protein may even downregulate inflammation and simultaneously promote tissue restoration. In mice receiving the combination of collagen peptide and whey protein, it was found that the tissue inflammation converges very early on (i.e. within days) towards the tissue repair process. The protective effect of the combination was sustained for at least two weeks. No beneficial effect was seen when collagen peptide and whey protein were used alone. Hence, it was found by the current inventors that the combined administration of collagen peptide and whey protein has synergetic and/or high therapeutic value.

In a clinical study, the current inventors confirmed the therapeutic value of combining collagen peptide and whey protein. In athletes undergoing strenuous exercise, the combination was found to downplay tissue inflammation, prevent tissue damage, and promote tissue repair. In addition, an improvement in clinical signs was observed, such as improved muscle and tendon functionality, reduced pain, and improved collagen repair. These improvements were strongly linked to a reduction in excessive inflammation (e.g. in muscle and tendon).

The mechanism by which collagen peptide and whey protein jointly achieve this effect is not known, however the current inventors show that the combination of collagen peptide and whey protein leads to a strong therapeutic effect, whereas this is not the case when collagen peptide or whey protein are used separately. This is indicative of a synergistic interaction between collagen peptide and whey protein.

In one aspect, the current disclosure describes a combination comprising collagen peptide (CP) and whey protein (WP) for use in ameliorating, preventing and/or treating tissue inflammation, a tissue inflammation-related condition, and/or a symptom of tissue inflammation and/or a tissue inflammation-related condition. This aspect of the current disclosure relates in particular to the therapeutic use of the combination to treat inflammation in skeletal tissues such as muscle, cartilage, bone, tendon, and/or ligament.

In one aspect, the current disclosure describes the use of the combination for ameliorating, preventing and/or treating inflammation induced by exercise, such as exercise overload, strength training, and/or sports.

In one aspect, the current disclosure describes the use of the combination for ameliorating, preventing and/or treating inflammation-related conditions including exercise-induced muscle damage, delayed onset muscle soreness, inflammatory myopathy tendinopathy, tendinitis, arthritis, polychondritis, osteomyelitis, ankylosing spondylitis, inflammation-mediated bone loss, and/or autoinflammatory bone disease.

In embodiments, collagen peptide and whey protein are provided as two or more separate formulations for the described use(s).

In embodiments, the collagen peptide and whey protein are provided in a single formulation such as a composition for the described use(s).

In embodiments, the collagen peptide and whey protein are provided in a formulation which is a food formulation, preferably a solid dosage form such as a powder for the described use(s).

In one aspect, the present disclosure describes a method of treating subjects experiencing tissue inflammation, a tissue inflammation-related condition, and/or or a symptom of tissue inflammation and/or a tissue-inflammation related condition using the combination and/or composition comprising collagen peptide and whey protein.

In one aspect, the present disclosure describes the use of the combination and/or composition comprising collagen peptide and whey protein for the manufacture of a medicament for the treatment of tissue inflammation, a tissue inflammation-related condition, and/or or a symptom of tissue inflammation and/or a tissue inflammation-related condition.

### Detailed description

The present invention relates to a combination comprising collagen peptide (CP) and whey protein (WP) for use in decreasing inflammation in skeletal muscle inflammation. The disclosure further describes the use in ameliorating, preventing and/or treating:
tissue inflammation; and/or
a tissue inflammation-related condition; and/or
a symptom of tissue inflammation and/or a tissue inflammation-related condition.

The use of the term "combination" in the current disclosure does not limit the combined use of collagen peptide and whey protein in the form of a physical union and/or as a composition. As used herein, "combination" refers to any combination form wherein collagen peptide and whey protein can attain the advantageous, preferably synergistic, effect according to the invention. To date, the current inventors have found that a synergistic effect of the combination of collagen peptide and whey protein is achieved when the collagen peptide and whey protein are administered to a subject not more than 24 hours separated from each other, however the synergistic effect of the combination of collagen peptide and whey protein may be greater when the collagen peptide and whey protein are administered to a subject not more than 12 hours separated from each other, preferably not more than 6 hours separated from each other. In some embodiments of the current invention, a joint effect may justify the combination of collagen peptide and whey protein present in single formulation. In other embodiments, a joint effect may justify the combination of collagen peptide and whey protein present in separate formulations. In yet other embodiments, a joint effect may justify the combination of collagen peptide and whey protein administered with a different timing and/or a different route of administration. For example, as part of the combination the current disclosure, the collagen peptide may be administered (e.g. to a human or animal subject) separately, sequentially or simultaneously to the whey protein. The collagen peptide and whey protein may be in a separate or in a same formulation, functional food, or solid dosage form as taught herein. The collagen peptide and whey protein may be presented side-by-side (e.g. to a human or animal subject) so that they can be applied simultaneously, separately or at intervals to said subject. In addition or alternatively, the collagen peptide and whey protein may be separately packed in the form of a "kit-of-parts". In addition or alternatively, the optimal administration scheme of collagen peptide and whey protein may be determined on a case-to-case basis, e.g. depending on the subject to be treated, depending on the condition or disease to be treated, depending on the route of administration, depending on the formulation, depending on the unit dose etc. As used herein, the term "treatment" or "treating" a disease or condition refers to a method of reducing or delaying such a condition before or after it has occurred. Treatment may be directed at one or more effects or symptoms of a disease or condition and/or the underlying pathology.

The treatment can be any reduction and can be, but is not limited to, the complete ablation of the disease or the symptoms of the disease. As compared with an equivalent untreated control, such reduction or degree of prevention is at least 5%, 10%, 20%, 40%, 50%, 60%, 80%, 90%, 95%, or 100% as measured by any standard technique.

As used herein, the term "prevent", "preventing", or "prevention" refers to a method for precluding, delaying, averting, or stopping the onset, incidence, severity, or recurrence of a disease or condition. For example, a method is considered to be a prevention if there is a reduction or delay in onset, incidence, severity, or recurrence of a disease or condition or one or more symptoms thereof in a subject susceptible to the disease or condition as compared to a subject not receiving the method. The reduction or delay in onset, incidence, severity, or recurrence can be about a 5, 10, 20, 30, 40, 50, 60, 70, 80, 90, 100%, or any amount of reduction in between.

As used herein, the term "subject" refers to any animal (e.g. a mammal), including, but not limited to humans, non-human primates, rodents, and the like, which is to be the recipient of a particular treatment. Typically, the terms "subject" and "patient" are used interchangeably herein in reference to a human subject.

The term "tissue inflammation" as used herein relates to a condition characterised by inflammation and/or signs of inflammation in the tissue, which may either be acute or chronic inflammation. The skilled person, such as the professional medical practitioner, is well-aware of the signs of inflammation and the best methods of measuring inflammation depending on the situation. For example, macroscopically, the five cardinal signs are heat, pain, redness, swelling, and loss of function (Latin: *calor, dolor, rubor, tumor,* and *functio laesa*)*.* An increased vascularization of the tissue may contribute to the latter signs of inflammation, tissue vascularization may be measured as an indication of inflammation. Inflammation may also be measured using biochemical markers, e.g. the local (e.g. in the tissue) or systemic (e.g. in the blood) expression of inflammation-associated cytokines (e.g. IL-6, IL-8, IL-β, IL-10, TNF-α, IL-17 and CCL-2). Inflammation may also be characterized by the molecular and cellular events in the blood and/or local tissue. For example, there generally is an altered activity of inflammation-associated transcription factors such as nuclear factor kappa-light-chain-enhancer of activated B cells (NF-κB), cyclooxygenase-2 (COX-2)/prostaglandin E(2) (PGE(2)), mitogen-activated protein kinase (MAPK), hypoxia-inducible factors-1 alpha (HIF-1α), and/or signal transducer and activator of transcription (STAT). Inflammation is generally also associated with a change in acute phase proteins, such as de C reactive protein, erythrocyte sedimentation rate, plasma viscosity, fibrinogen, and/or ferritin. Moreover, inflammation is usually correlated with a change local (e.g. in the tissue) or systemic (e.g. in the blood) number and composition of immune cells, e.g. neutrophils, macrophages, NK cells, mast cells, dendritic cells, T lymphocytes and/or B lymphocytes. A state of tissue inflammation may also be defined as any condition that typically is prone to treatment with an anti-inflammatory pain relievers such as glucocorticoids (e.g. cortisone), corticosteroids (e.g. prednisone), and/or non-steroidal anti-inflammatory drugs (NSAIDs such as aspirin, ibuprofen, or naproxen), e.g. as prescribed by a professional medical practitioner.

Since inflammation in a tissue is typically characterized by inflammation in the extracellular matrix (ECM) comprised within said tissue, the term "tissue" may be used interchangeably with the term "ECM" when the use of the combination as taught herein is disclosed.

In an embodiment, the present disclosure describes a combination comprising collagen peptide (CP) and whey protein (WP) for use in ameliorating, preventing and/or treating:
- ECM inflammation; and/or
- an ECM inflammation-related condition; and/or
- a symptom of ECM inflammation and/or an ECM inflammation-related condition.

The term "tissue inflammation-related condition" as used herein refers herein to a condition arising in a subject which is characterized by and/or caused by tissue inflammation. The tissue inflammation-related condition as used herein is typically a medical condition and/or associated with symptoms, typically symptoms at the site of tissue inflammation. In addition or alternatively, the term condition as used herein may also be used interchangeably herein with the term "disease" or "pathology". The tissue inflammation-related condition as used herein is preferably one or more of: exercise-induced muscle damage, delayed onset muscle soreness, inflammatory myopathy, tendinopathy, tendinitis, arthritis, polychondritis, osteomyelitis, ankylosing spondylitis, inflammation-mediated bone loss, and/ autoinflammatory bone disease, or a related condition thereof.

The term "symptom" as used herein relates to any physical (e.g. a reduced function), mental (e.g. pain), or biological feature (e.g. a biochemical or histological change detectable in a bodily tissue) which is regarded as indicating a condition of disease, typically a pathological state or process. A symptom may be experienced by a subject having the symptom, or may be diagnosed or measured as part of a diagnostic process.

The current inventors found that the combination of collagen peptide and whey protein as taught herein provides effective immune-modulation to alleviate, prevent or treat skeletal diseases or conditions wherein there is a disturbed inflammatory response. Inflammation may play a crucial role in certain skeletal diseases, and wherein the therapeutic targeting of inflammation may alleviate, prevent or cure disease. Such diseases or conditions typically do not heal by simples rest or by natural mechanisms (e.g. a therapeutic intervention is preferably needed). In addition, non-immunological (therapeutic) strategies may be insufficient in their treatment or prevention.

Current interventions attempting for alleviating the inflammatory component in inflammatory skeletal disease mainly at aim at reducing the symptoms of disease and are usually pharmacological in nature. Examples include the use of anti-inflammatory pain relievers such as glucocorticoids (e.g. cortisone), corticosteroids (e.g. prednisone), non-steroidal anti-inflammatory drugs (NSAIDs such as aspirin, ibuprofen, or naproxen), and narcotic pain relievers (Schoenfeld et al. Sports Med. 2012 Dec 1;42(12):1017-28, Dinarello et al. Cell. 2010 Mar 19;140(6):935-50).

Despite the many anti-inflammatory pain relievers available, their use in the treatment of inflammatory skeletal diseases leads to inadequate therapeutic effects. First, traditional anti-inflammatory drugs generally reduce the symptoms of inflammation, but are not aimed at skeletal tissue repair, that is, they do not simultaneously target tissue, extracellular matrix) ECM and/or collagen remodelling and repair. Second, pharmacological drugs may lead to comorbidity, side effects, and therefore are typically not suitable for widespread, prolonged usage. Finally, there are concerns about possible interfering effects of anti-inflammatory on the skeletal tissue repair process. To illustrate, there is emerging evidence that the action of NSAIDs, which are routinely prescribed for treatment of exercise-induced muscle damage (EIMD), may impair normal skeletal muscle growth in response to functional overload (Schoenfeld et al. Sports Med. 2012 Dec 1;42(12):1017-28). Moreover, the modification of inflammation in tendinitis i.e. with the use of steroids and NSAIDs could also alter the natural healing cascade, providing short-term relief but leading to further pathology (Charnoff et al. 2017, "Tendinosis (Tendinitis)". StatPearls. Treasure Island (FL): StatPearls Publishing).

On the one hand, the combination as taught herein may reduce the reliance on pharmacological anti-inflammatory pain relievers or toxic agents. Considering the high safety profile of collagen peptide and whey protein, the combination can be used more continuously, i.e. on a daily basis or as a preventive approach. On the other hand, the combination as taught herein may be more effective than pharmacological anti-inflammatory pain relievers or toxic agents, as the combination both downplays the unwanted inflammatory component of the disease and promotes tissue remodelling and/or repair.

Particular (skeletal) conditions, such as particular (skeletal) tissue inflammation-related conditions, may benefit most from the combination as part of the current disclosure. For example, inflammation plays an important role in exercise-induced muscle damage (EIMD). Several lines of evidence indicate that prolonged and/or high-intensity exercise (e.g. exercise overload) leads to excessive muscle and tendon inflammation in exercise-induced muscle damage, which may typically lead to tissue damage, loss, or mitigated repair or function, among others (Cerqueira et al. Front Physiol. 2020 Jan 9;10:1550, Hennigar et al. FASEB J . 2017 Sep;31(9):3719-3728, Zdzieblik et al. Nutrients. 2021 Feb; 13(2): 523). In contrast, inflammation does not seem to play a role in disuse muscle atrophy. Consequently, the combination as taught herein may be more effective in ameliorating, preventing, and/or treating exercise-induced muscle damage than disuse muscle atrophy. Conversely, non-immunological strategies may be superior in ameliorating, preventing, and/or treating ameliorating, preventing, and/or treating than exercise-induced muscle damage.

Sarcopenia is also a common example of a disease of the skeletal muscle tissue, wherein the disease is typically characterized by involuntary muscle loss (muscle atrophy). The muscle atrophy in sarcopenia often occurs with aging and/or immobility, although here may be other causes of sarcopenia. For example, different types of sarcopenia include age-related sarcopenia, immobilisation-related sarcopenia, stress-related sarcopenia, and/or unbalanced diet-related sarcopenia. Inflammation may not play a role in (all types) of sarcopenia and/or may not be diagnosable as inflammation-related therein. Hence, the pathogenesis of the disease may be different from the tissue inflammation-related conditions the combination is more preferably used for. In an embodiment, the use of the combination as disclosed herein excludes the use for (types of) sarcopenia wherein inflammation is not part of disease onset, disease progression, and/or wherein inflammation is not a symptom. In an embodiment, the use of the combination as disclosed herein includes the use for (types of) sarcopenia, wherein inflammation is part of disease onset, disease progression, and/or wherein inflammation is a symptom.

Endurance performance is a condition typically characterised by a reduction in mitochondrial activity in a person or animal. In endurance performance, an important factor in aerobic capacity is oxygen absorption. Inflammation may not play a role in (all types) of endurance performance, especially in conditions when the performance is mostly limited by an insufficient aerobic metabolism or oxygen absorption needed to supply the musculature with the required energy in the form of ATP (adenosine triphosphate) over a long period. Moreover, inflammation may not be present and/or diagnosable in (all types) of endurance performance. Other, non-immunological strategies may be more effective in improving endurance performance. In an embodiment, the use of the combination as disclosed excludes the use for endurance performance.

In the (skeletal) conditions, such as the (skeletal) tissue inflammation-related conditions as taught herein targeted by the current disclosure, a prolonged or exaggerated exposure of the tissue to inflammatory cues may be a trigger for the induction of matrix metalloproteinase (MMP) expression and activation of MMP enzymes. MMPs are key effectors in ECM breakdown, repair, and remodelling. In physiological processes or mild injuries, the elevation in MMP activity that occurs as part of the inflammatory phase of healing is likely beneficial in the repair and regeneration of damaged ECM. However, prolonged or excessive inflammatory conditions, a persistent increase in MMP activity likely leads to disrupted ECM ultrastructure, collagen breakdown and impaired skeletal tissue function.

In an embodiment, the present disclosure describes the combination as taught herein for use in modulating inflammation, preferably decreasing inflammation.

In several of the embodiments of the current disclosure, the combination as part of the current disclosure may be used to modulate (e.g. increase or decrease) any one or more of the signs of inflammation, e.g. one or more of the signs of inflammation as disclosed herein. The sign of inflammation may for example be one or more of the macroscopic signs of inflammation as taught herein, increased vascularization as taught herein, one or more of the biochemical changes as taught herein, one or more of the molecular and cellular events as taught herein, one or more of the changes in transcription factors as taught herein, one or more of the changes in acute phase proteins as taught herein, and/or the number and composition of immune cells as taught herein. However, the current disclosure is not limited to the signs of inflammation disclosed herein.

In an embodiment, the use of the combination as part of the current disclosure is directed to reducing a pro-inflammatory response. In addition or alternatively, the use of the combination as part of the current disclosure is directed to enhancing an anti-inflammatory response.

In a preferred embodiment, the current disclosure relates to the use of the combination as taught herein for use in ameliorating, preventing and/or treating tissue inflammation and/or a tissue inflammation-related condition, wherein the tissue is skeletal tissue, preferably skeletal muscle, cartilage, bone, tendon, and/or ligament.

The skeletal tissue as used herein may in addition or alternatively comprise the connective tissue covering and/or connected to the skeletal tissue, such as the connective tissue covering the skeletal muscle, cartilage, bone, tendon, and/or ligament, including, but not limited to epimysium, muscle fascia, synovial membrane, cartilage fascia, bone fascia, endosteum, periosteum.

In a preferred embodiment, the present disclosure describes the combination for use in ameliorating, preventing and/or treating a tissue inflammation and/or a tissue inflammation-related condition induced by exercise.

In an embodiment, the present disclosure describes the combination for use in ameliorating, preventing and/or treating tissue inflammation induced by exercise overload, strength training, and/or sports.

In a preferred embodiment, the present disclosure describes the combination for use in tissue inflammation and/or a tissue-inflammation related condition that is induced by exercise. The term "exercise" as used herein relates to any physical activity that can be planned, structured, and repetitive and has as a final or an intermediate objective. The term "exercise" as used herein typically has as goal the improvement or maintenance of physical fitness. For example, "exercise" may include all professional or non-professional activities including: sports, strength or resistance training, and endurance or aerobic activities. In addition or alternative, "exercise" may include endurance training. The term endurance training generally refers to training the aerobic system as opposed to the anaerobic system, e.g. by performing physical exercise at an increased heart rate for a prolonged period of time (e.g. at 70% maximum heart rate (MHR) for at least 30 minutes), for example during walking, running, jogging, cycling, swimming, rope jumping etc. Inflammation may not play a role in (all types) of exercise, especially in exercise with low-impacting exercise and/or exercise not involving high strains and/or forces applied on the tissue. In addition or alternatively, inflammation may also not play a role in exercise which is non-repetitive. In an embodiment, the use of the combination as taught herein excludes the use for exercise wherein inflammation is not a symptom or is not diagnosable.

The term "exercise overload" as used herein relates to an overload of the tissue as caused by high strains and/or forces applied on the tissue during any form of physical exercise. The overload is typically caused by acute overload, however it may also be caused by repetitive overload. Exercise overload, foremost acute overload, typically leads to high strains on the skeletal tissue, causing microdamage to the architecture of the skeletal tissue. In addition, or alternatively, exercise overload generally results in local inflammatory responses and tissue inflammation-related symptoms (e.g. reduced skeletal tissue function, reduced skeletal tissue repair, skeletal tissue pain). In an embodiment, the present disclosure describes the combination as taught herein for use in ameliorating, preventing and/or treating tissue inflammation induced by exercise overload, more preferably exercise overload caused by acute overload. Example of exercise overload include mostly exercises wherein there is a high strain applied on the skeletal muscle and connective tissues, such as involving (heavy) jumps and squats.

In an embodiment, the present disclosure describes the combination for use in ameliorating, preventing and/or treating exercise-induced muscle damage, delayed onset muscle soreness, inflammatory myopathy, tendinopathy, tendinitis, arthritis, polychondritis, osteomyelitis, ankylosing spondylitis, inflammation-mediated bone loss, and/or autoinflammatory bone disease, or related conditions.

The term "exercise-induced muscle damage" (EIMD) as used herein refers to muscle damage that occurs due to exercise involving eccentric (muscle lengthening) actions and which typically results in structural damage to the myofibrils and surrounding extracellular matrix (ECM). EIM can be seen for example in novel or unaccustomed exercise, including in athletes. EIM generally is associated with a (temporary) decrease in muscle force production, a rise in passive tension, an increase in muscle soreness and swelling, and/or an increase in intramuscular proteins in blood. A number of animal and human studies have reported an increase in inflammation and muscle collagen turnover in EMD (e.g. < 72 h following muscle-damaging exercise, indicative of extensive degradation and remodelling in the ECM (Miller et al. J Physiol 567(3):1021-1033), therefore inflammation-modulating strategies may be particularly effective. In a preferred embodiment, the combination as disclosed herein is used for the alleviation, prevention and/or treatment of EIMD.

The term "delayed onset muscle soreness" (DOMS) as used herein refers to the pain and stiffness felt in muscles several hours to days after unaccustomed or strenuous exercise. The pain is typically felt only when the muscle is stretched, contracted or put under pressure, but not when it is at rest. Although there generally is variance among exercises and individuals, DOMS usually increases in intensity in the first 24 hours after exercise, peaks from 24 to 72 hours, then subsides and disappears up to seven days after exercise. DOMS is presumed to be caused by eccentric exercise, that is, exercise consisting of eccentric (lengthening) contractions of the muscle, e.g. as in EIMD. The acute but delayed nature of DOMS is considered to be indicative of an inflammatory immune response, therefore inflammation-modulating strategies may be particularly effective in its prevention/treatment (MacIntyre et al. Sports Med . 1995 Jul;20(1):24-40). In a preferred embodiment, the combination as disclosed herein is used for the alleviation, prevention and/or treatment of DOMS.

The term "inflammatory myopathy" as used herein refers to a disease featuring weakness and inflammation of muscles and in some types muscle pain. The four main types of inflammatory myopathy are generally considered to be polymyositis (i.e. which affects skeletal muscles) dermatomyositis (i.e. which includes a skin rash and progressive muscle weakness, inclusion body myositis (i.e. which is characterized by progressive muscle weakness and shrinkage) and necrotizing autoimmune myopathy (i.e. which includes weakness in the upper and lower body, difficulty rising from low chairs or climbing stairs, fatigue, and muscle pain). An inflammatory myopathy often is an autoimmune disorder. Inflammatory infiltrates are typically seen in muscle cells in inflammatory myopathy, therefore inflammation-modulating strategies may be particularly effective in its prevention/treatment. In a preferred embodiment, the present disclosure describes the combination used for the alleviation, prevention and/or treatment of an inflammatory myopathy, more preferably polymyositis, dermatomyositis, inclusion body myositis, and/or necrotizing autoimmune myopathy.

The term "tendinopathy" as used herein refers to a group of tendon disorder that typically results in pain, swelling, and/or impaired function. It most commonly occurs around the shoulder (rotator cuff tendinitis, biceps tendinitis), elbow (tennis elbow, golfer's elbow), wrist, hip, knee (jumper's knee, popliteus tendinopathy), or ankle (Achilles tendinitis), and generally is typically worse with movement. Athletes are generally at higher risk of developing tendinopathy, as they may experience trauma, overloading, or excessive use of the tendon. Inflammation appears to play a role in tendonitis, e.g. both inflammation and damage-induced matrix remodelling markers are known to be expressed in the tendon following exercise overload (Spiesz et al. J Orthop Res. 2015 Jun;33(6):889-97). Hence, inflammation-modulating strategies may be particularly effective in preventing/treating tendinopathy. In a preferred embodiment, the present disclosure describes the combination used for the alleviation, prevention and/or treatment of tendinopathy.

The term "tendinitis" as used herein refers to an inflammation of the tendon. Complications in tendinitis may include contractures of the tendon with reduced tendon liability, tendon adhesions, atrophy of muscles, loss of functionality, even up to and including disability. If left untreated tendinosis can result in tendon rupture. The inflammatory response in tendinitis is most frequently caused by acute overload, repetitive stress, or chemical irritation. Tendinitis injuries may plague a wide variety of patients, ranging from vigorous athletes to non-athletes.

Tendinitis is typically also a hallmark of spondyloarthritis. Tendinitis, by definition, implies that tendon injury is accompanied by an inflammatory response, therefore inflammation-modulating strategies may be particularly effective in preventing/treating tendinopathy. In a preferred embodiment, the present disclosure describes the combination used for the alleviation, prevention and/or treatment of tendinitis.

The term "arthritis" as used herein refers to joint inflammatory diseases, which generally is associated with symptoms such as joint pain and stiffness. In the current disclosure, the term "arthritis" refers to all subsets of arthritis, including osteoarthritis, rheumatoid arthritis, psoriatic arthritis, ankylosing spondylitis, gout and systemic lupus erythematosus. Considering the role of catabolic and/or inflammatory factors in arthritis, inflammation-modulating strategies may be particularly effective in preventing/treating different forms of arthritis. In a preferred embodiment, the combination as disclosed herein is used for the alleviation, prevention and/or treatment of arthritis, more preferably osteoarthritis, rheumatoid arthritis, gout, psoriatic arthritis, reactive arthritis, and/or septic arthritis, most preferably osteoarthritis. Loss of cartilage is a hallmark in osteoarthritis. In osteoarthritis, the cartilage is easily attacked by proinflammatory factors and oxidative stress since the cartilage tissue is avascular, which leads to cartilage breakdown (Zhang et al. Tissue Eng Part B Rev. 2014 Dec 1; 20(6): 655-668).

The term "inflammation-mediated bone loss" as used herein relates to any condition or disease associated with bone loss (osteolysis) which is would be considered (by a professional medical practitioner) as pathological. Examples up inflammation-mediated bone loss which can be targeted as part of the current disclosure include, but are not limited to inflammatory bowel disease, chronic obstructive lung disease, cystic fibrosis, and/or periodontitis, rheumatoid arthritis (Redlich et al. Nat Rev Drug Discov. 2012 Mar 1;11(3):234-50).

In another preferred embodiment, the present disclosure describes the combination for use in ameliorating, preventing tissue inflammation and/or a tissue-inflammation-related condition that is catabolic. The term "catabolic" herein relates to a state wherein the catabolic response (tissue degradation) predominates over the anabolic response (tissue formation/repair), which typically leads to a loss of tissue mass. At the molecular level, this generally is associated with an excessive breakdown of ECM molecules, including collagen, as compared to a healthy, normal state. In addition or alternatively, the term "catabolic" may therefore also refer to a disruption of the balance between ECM protein (e.g. collagen) synthesis and breakdown, for example characterized by an increased activity of tissue-degrading enzymes such as MMPs. The skilled person is well-aware of the methods to measure or diagnose a catabolic state of the tissue, which is dependent on the specific tissue of interest.

In a preferred embodiment, the present invention relates to a combination comprising collagen peptide and whey protein for use in decreasing inflammation in skeletal muscle inflammation, wherein the skeletal muscle inflammation is characterised by one or more symptoms selected from the group consisting of:
- reduced skeletal muscle tissue function;
- reduced skeletal muscle tissue repair;
- skeletal muscle tissue loss;
- skeletal muscle tissue soreness; and/or
- skeletal muscle tissue pain.

In an embodiment, the present disclosure describes the symptom is one or more selected from the group consisting of:
- reduced tissue function, preferably reduced skeletal tissue function, more preferably reduced skeletal muscle tissue and/or reduced tendon tissue function;
- reduced tissue repair, preferably reduced skeletal tissue repair, more preferably reduced skeletal muscle tissue repair and/or reduced tendon tissue repair;
- tissue loss, preferably skeletal tissue loss, more preferably skeletal muscle tissue loss and/or tendon tissue loss;
- tissue soreness, preferably skeletal tissue soreness, more preferably skeletal muscle tissue soreness and/or tendon tissue soreness;
- tissue pain, preferably skeletal tissue pain, more preferably skeletal muscle tissue pain and/or tendon tissue pain;
- excessive collagen turnover; and
- excessive extracellular matrix (ECM) cleavage.

In addition or alternatively, the present disclosure describes a symptom of tissue inflammation and/or tissue inflammation-related condition which may be one or more selected from the group consisting of:
- reduced muscle force;
- reduced jump performance; and
- reduced knee-extension force.

In an embodiment, the present disclosure describes the combination as taught herein used to reduce pain in a subject, such as pain related to tissue inflammation and/or a tissue inflammation-related condition, wherein the reduction in pain may for example be characterised by a reduction in pain perception according to any one more of the validated measure for pain, e.g. the visual analog scale (VAS), Verbal Numerical Rating Scale (VNRS), Verbal Descriptor Scale (VDS), Brief Pain Inventory, Wong-Baker rating scale, Wharton Impairment and Pain Scale, or the VISA-P scale.

In another embodiment, the present disclosure describes the combination as taught herein used to improve the muscular functional capacity in a subject. The skilled person is well-aware of the validated methods to assess muscular functional capacity. For example, an improved muscular functional capacity may be demonstrated by countermovement jump-performance or a squat jump test, e.g. measured on a force platform. In addition or alternatively, an enhanced muscular functional capacity may be demonstrated by measuring the maximal isometric knee-extension force, e.g. assessed on an isokinetic dynamometer.

In an embodiment, the present disclosure describes the use of the combination as part of the current disclosure used for increasing jump performance by a subject and/or increasing the maximal isometric knee-extension force in a subject.

In an embodiment, the present disclosure describes the combination as taught herein used to improve the patellar tendon integrity in a subject. The skilled person is well-aware of the validated methods to assess tendon integrity. For example, an improved tendon integrity may be demonstrated by (power color doppler) ultrasound, e.g. including the assessment of patellar tendon thickness and/or neovascularization which can be scored, for example followed by calculation of the Ohberg score.

In an embodiment, the present disclosure describes the combination as taught herein used to reduce excessive collagen turnover, e.g. increased collagen turnover relative to a normal, healthy state. The skilled person is well-aware of the validated markers and methods to determine excessive collagen turnover. For example, accelerated collagen turnover may be determined based on levels of one or more markers of collagen turnover, e.g. carboxyl-terminal peptide of procollagen type I (PIP), carboxyl-terminal telopeptide of collagen type I (CITP), and amino-terminal peptide of procollagen type III (PIIINP).

In an embodiment, the present disclosure describes the combination as taught herein used to reduce excessive ECM cleavage, e.g. increased ECM cleavage relative to a normal, healthy state. The skilled person is well-aware of the validated markers and methods to determine ECM cleavage. For example, accelerated collagen turnover may be determined based on levels of markers of one or more of endostatin, tumstatin, canstatin, arresten and hexastatin, and endorepellin. In addition or alternatively, the levels of proteases such as metalloproteinases (MMPs) may be indicative of a reduction in excessive ECM cleavage.

The term "collagen peptide" (CP) as used herein refers to a peptide derived from collagen. collagen peptides are short chains of amino acids typically extracted from native (full-length) collagen such as via enzymatic hydrolysis (also called enzymatic hydrolysation). The degree of hydrolysis usually has an impact on the average molecular weight of the final product. The term "collagen peptide" may be used interchangeably and synonymous with the terms "hydrolysed collagen" or "collagen hydrolysate".

In a preferred embodiment, the collagen peptide as part of the current invention is derived from collagen obtained from animal skin, cartilage, bone, and/or connective tissue.

In a preferred embodiment, collagen peptide as part of the current invention is derived from porcine, bovine and/or fish collagen.

The collagen as disclosed herein is preferably derived from collagen type I and/or collagen type II.

The collagen peptide as taught in the current disclosure may comprise collagen which is hydrolysed or partially hydrolysed, wherein the collagen may be derived from any species of animal. In addition or alternatively, an animal herein may refer to any animal capable of providing connective tissue, and the connective tissue is used to prepare collagen peptide.

In a preferred embodiment, the collagen as taught herein is derived from a cow. In another preferred embodiment, the collagen as taught herein is derived from a pig. In yet another preferred embodiment, the collagen as taught herein is derived from a fish.

In various embodiments, the collagen as provided in the combination of the current disclosure is a mixture of collagen from different sources, such as collagen originating from multiple animal species, and/or collagen originating from different tissues. For example, the collagen as provided in the combination of the current invention may be a mixture of collagen chosen from fish collagen, porcine collagen, and bovine collagen. In addition or alternatively, the collagen may be a mixture of collagen chosen from skin-, cartilage-, bone-, and/or connective tissue-derived collagen.

The connective tissue as taught herein refers to a group of tissues present between various tissues, organs and cells, including but not limited to being present in the corpus callosum, skin, spines (e.g. antler), protrusions (e.g. humps, lion's milk), horns, head, brain, neck, ear, eye, nose, tongue, lip, mouth, oesophagus, trachea, limbs, feet, toes, palms, claws, bones, cartilage, bone marrow, joints, membranes, hind, ligaments, tendons, tendons interval, tendon, rib, diaphragm, muscle, skeletal muscle, smooth muscle, intestine, oesophagus, trachea, venetian, belly, blood vessels, bladder, stomach, aorta, heart, liver, kidney, chest, lung, spleen, pancreas, egg, sperm, testis, ovary, nerve, gallbladder, belly.

In a preferred embodiment, the collagen as taught herein is derived from the skin and/or skin connective tissue. In another preferred embodiment, the collagen as taught herein is derived from the hind. In another preferred embodiment, the collagen as taught herein is derived from cartilage. In yet another preferred embodiment, the collagen as taught herein is derived from bone.

In various embodiments of the current disclosure, the collagen peptide may be produced by the enzymatic hydrolysis or partial enzymatic hydrolysis of collagen, wherein the enzyme used for this purpose may be one or more selected from the group consisting of serine protease, alkaline protease, neutral protease, flavour protease, complex protease, thiol protease, bromelain, metalloprotease, aspartame, protease, carboxypeptidase, pepsin, chymotrypsin, trypsin, cathepsin K, chymotrypsin, papain, and subtilisin combination.

The term "whey protein" (WP) as used herein refers to a protein or mixture of proteins isolated from whey, the liquid material created as a by-product of cheese production. The proteins usually comprise of α-lactalbumin, β-lactoglobulin, serum albumin and immunoglobulins. whey protein is most frequently produced as a left over when milk is coagulated during the process of cheese production. Processing can for example be done by simple drying, or the relative protein content can be increased by removing the lactose, lipids and other non-protein materials. Whey can be classified into sweet whey and acid whey. Sweet whey is the byproduct of rennet-coagulated cheese, and typically has a pH greater than or equal to 5.6. Acid whey (also called sour whey) is the byproduct of acid-coagulated cheese and typically has a pH less than or equal to 5.1.

The whey protein disclosed as part of the combination as taught herein may comprise or be comprised in any type of whey protein, but preferably is or is comprised in one or more of whey protein concentrate (WPC), whey protein isolate (WPI), whey protein hydrolysate (WPH), and native whey protein. The skilled person is well-aware of these and other types of whey protein that are highly consumed (Lagrange et al. J Food Sci. 2015 Mar;80 Suppl 1:A16-22), and that are suitable as part of the combination as taught herein.
whey protein concentrate (WPC) refers herein to a whey protein which typically has a low (but still significant) level of fat and cholesterol but, in general, compared to other forms of whey protein, are higher in carbohydrates in the form of lactose. They are generally 29%-89% protein by weight. Whey protein isolate (WPI) refers herein to a whey protein which is processed to remove the fat and lactose. They are generally 90%+ protein by weight. Like WPC, WPI is mild to slightly milky in taste. whey protein hydrolysate (WPH) refers herein to a whey protein that is pre-digested and partially hydrolysed for the purpose of easier metabolizing. Native whey protein refers herein to a whey protein which is extracted from skim milk, not a by-product of cheese production, and produced as a concentrate and isolate.

The current inventors found a synergistic effect between collagen peptide and whey protein concentrate containing 70 wt.% acid whey. In addition, the current inventors found protective effects of collagen peptide and whey protein isolate on inflammation and tissue damage in subjects undergoing strenuous exercise, wherein the whey protein isolate comprised 80 wt.% sweet whey.

In a preferred embodiment, the whey protein in the combination as part of the current invention is acid whey or sweet whey.

In a preferred embodiment, the whey protein in the combination as part of the current invention is provided as a whey protein isolate or a whey protein concentrate. In addition or alternatively, the whey protein may be or be comprised in a whey protein isolate or a whey protein concentrate.

In various embodiments, the whey protein concentrate, whey protein isolate, whey protein hydrolysate as taught herein has a whey content of at least 70 wt.%, preferably at least 80 wt.%, more preferably at least 90 wt.%, most preferably at least 95 wt.%.

In a preferred embodiment of the current invention, the collagen peptide has a mean molecular weight (M_{W}) between 1500 Da and 6000 Da.

The molecular weight as disclosed herein is preferably the weight averaged molecular weight Mw.

In a more embodiment of the current invention, the collagen peptide has a mean molecular weight between 1500 Da and 3000 Da or a mean molecular weight between 3000 Da and 6000 Da.

In certain embodiments of the combination as part of the current disclosure, the collagen peptide has a mean molecular weight between 300 Da and 10000 Da, e.g. between 500 Da and 7500 Da, between 1000 Da and 6000 Da, between 1500 Da and 5000 Da, or between 1500 Da and 3000 Da.

In an embodiment, the collagen peptide as taught herein has a mean molecular weight between 1250 Da and 2750 Da, preferably between 1500 Da and 2500 Da, more preferably between 1750 Da and 2250 Da.

In an embodiment, the collagen peptide as taught herein has a mean molecular weight of between 4250 Da and 5750 Da, preferably between 4500 Da and 5500 Da, more preferably between 4750 Da and 5250 Da.

The collagen peptide as taught herein may have a protein content of 75-99 wt.%, preferably 80-99 wt.%, more preferably 85-99 wt.%, most preferably 90-99 wt.%.

In an embodiment, the collagen peptide as part of the combination as taught herein comprises glycine in an amount of 15-25 wt.%, proline in an amount of 9-13 wt.%, and hydroxyproline in an amount of 9-13 wt.%, all per total weight of the amino acids in the collagen peptide.

In an embodiment, the collagen peptide as part of the combination as taught herein comprises glycine in an amount of 5-50 wt.%, preferably in an amount of 10-30 wt.%, more preferably in an amount of 12.5-27.5 wt.%, even more preferably in an amount of 15-25 wt.%, most preferably in an amount of 17.5-22.5 wt.%, all per total weight of the amino acids in the collagen peptide.

In an embodiment, the collagen peptide as part of the combination as taught herein comprises proline in an amount of 2-20 wt.%, preferably in an amount of 7-15 wt.%, more preferably in an amount of 8-14 wt.%, even more preferably in an amount of 9-13 wt.%, most preferably in an amount of 10-12 wt.%, all per total weight of the amino acids in the collagen peptide.

In an embodiment, the collagen peptide as part of the combination as taught herein comprises hydroxyproline in an amount of 2-20 wt.%, preferably in an amount of 7-15 wt.%, more preferably in an amount of 8-14 wt.%, even more preferably in an amount of 9-13 wt.%, most preferably in an amount of 10-12 wt.%, all per total weight of the amino acids in the collagen peptide.

In an embodiment, the collagen peptide as part of the combination as taught herein has a molecular weight of 1750-2250 Da and comprises glycine in an amount of 17.5-22.5 wt.%, proline in an amount of 10-12 wt.%, and hydroxyproline in an amount of 10-12 wt.%, all per total weight of the amino acids in the collagen peptide.

In an embodiment, the collagen peptide as part of the combination as taught herein has a molecular weight of 4750-5250 Da and comprises glycine in an amount of 17.5-22.5 wt.%, proline in an amount of 10-12 g wt.%, and hydroxyproline in an amount of 10-12 g wt.%, all per total weight of the amino acids in the collagen peptide.

In an embodiment, the collagen peptide as part of the combination as taught herein has a molecular weight of between 1500-3000 Da and comprises glycine in an amount of 17.5-22.5 wt.%, proline in an amount of 10-12 wt.%, and hydroxyproline in an amount of 10-12 wt.%, all per total weight of the amino acids in the collagen peptide.

In an embodiment, the collagen peptide is present in the combination in an amount of between 0.5 g and 200 g, preferably between 1 g and 100 g, more preferably between 2 g and 50 g, most preferably between 5 g and 25 g, wherein the amount is the dry weight amount per unit dose.

The term "unit dose" as used herein relates to an amount or unit of e.g. a compound, substance, formulation, dosage form, or composition taken by a patient (and/or administered to a patient) in a single dose. The unit dose may for example be in a pre-prepared form (e.g. prepacked dosage) ready for administration to a subject. The unit dose may for example (also) be identifiable from the product packaging or label. For example, the total daily dose may be divided into multiple unit doses each having a reduced dose as compared to the total daily dose.

The terms "administer" or "administration" as used herein relate to the act of providing a substance, composition and/or combination (typically a therapeutic substance, composition and/or combination) to a subject consuming it. A subject consuming a certain substance, composition and/or combination may administer the substance, composition and/or combination to himself/herself/itself. In such a case the terms "administered to" may be interpreted as "taken by" or "consumed by".

In a preferred embodiment of the current disclosure, the ratio of collagen peptide and whey protein (CP:WP) is between 5:1 and 1:5, preferably between 1:1 and 1:5, more preferably between 1:1 and 1:4, most preferably between 1:1 and 1:3. The CP:WP ratio as taught herein is preferably based on the total dry weight amount administered to a subject per day and/or the dry weight amount per unit dose.

In a preferred embodiment, the collagen peptide is present in the combination in a daily amount of between 0.5 g and 200 g, preferably between 1 g and 100 g, more preferably between 2 g and 50 g, most preferably between 5 g and 25 g, and/or the whey protein is present in the combination in a daily amount of between 0.1 g and 200 g, preferably between 1 g and 100 g, more preferably between 5 g and 75 g, most preferably between 15 g and 50 g, wherein the daily amount is the total dry weight amount administered to a subject per day.

In a preferred embodiment, the collagen peptide is present in the combination in an amount of between 0.5 g and 200 g, preferably between 1 g and 100 g, more preferably between 2 g and 50 g, most preferably between 5 g and 25 g, and/or the whey protein is present in the combination in an amount of between 0.1 g and 200 g, preferably between 1 g and 100 g, more preferably between 5 g and 75 g, most preferably between 15 g and 50 g, wherein the amount is the dry weight amount per unit dose.

In addition or alternatively, the amount of the collagen peptide and/or whey protein in the combination according to the present disclosure is set preferably at between 1.5 mg and 1500 mg, preferably between 7 mg and 700 mg, more preferably between 15 mg and 360 mg, most preferably between 15 mg and 215 mg, which is on a dry weight basis of the total combination, wherein the amount is the total amount administered to a subject per day per kg body weight of the subject and/or the amount administered in a single dosage form per kg body weight of the subject.

In a preferred embodiment of the current disclosure, the combination is provided in two or more separate dosage forms. Preferably, the two or more separate dosage forms contain independently collagen peptide, independently whey protein, or a combination of collagen peptide and whey protein. The collagen peptide or whey protein may be present in the separate dosage form as taught herein in an amount of 50-100 wt.%, preferably 70-100 wt.%, more preferably 80-100 wt.%, even more preferably 90-100 wt.%, most preferably 95-100 wt.%, wherein the wt.% is based on the total weight of the dry components of said formulation.

In a preferred embodiment of the current disclosure, the combination is provided in a single dosage form, e.g. as a composition. The collagen peptide and whey protein may be present in the single dosage form as taught herein in an amount of 25-50 wt.%, preferably 35-50 wt.%, more preferably 40-100 wt.%, even more preferably 45-100 wt.%, most preferably 47.5-50 wt.%, wherein the wt.% is based on the total weight of the dry components of said formulation.

The term 'composition' as used herein refers to the final dosage form in which collagen peptide and whey protein are both incorporated when the combination is administered simultaneously in a single composition. The composition possibly comprises additional ingredients, e.g. to improve the uptake and/or bioactivity of the collagen peptide and/or whey protein. The term 'composition' may be used also interchangeably with the term 'single dosage form'.

In an embodiment of the current disclosure, the daily amount of collagen peptide as taught herein is provided as:
- 5 separate units comprising collagen peptide, each unit providing a dose of collagen peptide corresponding on average to a fifth of the daily amount of collagen peptide and/or each unit providing a dose of collagen peptide of 100 mg - 40 g;
- 4 separate units comprising collagen peptide, each unit providing a dose of collagen peptide corresponding on average to a fourth daily amount of collagen peptide, and/or each unit providing a dose of collagen peptide of 125 mg - 50 g;
- 3 separate units comprising collagen peptide, each unit providing a dose of collagen peptide corresponding on average to a third of the daily amount of collagen peptide, and/or each unit providing a dose of collagen peptide of 167 mg - 67 g ;
- 2 separate units comprising collagen peptide, each unit providing a dose of collagen peptide corresponding on average to half of the daily amount of collagen peptide, and/or each unit providing a dose of collagen peptide of 250 mg -100 g; or
- 1 single unit comprising collagen peptide, the unit providing a dose of collagen peptide corresponding to the daily amount of collagen peptide, and/or the unit providing a dose of collagen peptide of 500 mg - 200 g.

In an embodiment of the current disclosure, the daily amount of whey protein is provided as:
- 5 separate units comprising whey protein, each unit providing a dose of whey protein corresponding on average to a fifth of the daily amount of whey protein, and/or each unit providing a dose of whey protein of 20 mg - 40 g ;
- 4 separate units whey protein, each unit providing a dose of whey protein corresponding on average to a fourth of the daily amount of whey protein, and/or each unit providing a dose of whey protein of 25 mg - 50 g;
- 3 separate units whey protein, each unit providing a dose of whey protein corresponding on average to a third of the daily amount of whey protein, and/or each unit providing a dose of whey protein of 33 mg - 67 g;
- 2 separate units whey protein, each unit providing a dose of whey protein corresponding on average to half of the daily amount of whey protein, and/or each unit providing a dose of whey protein of 50 mg -100 g; or
- 1 single unit whey protein, the unit providing a dose of whey protein corresponding to the daily amount of whey protein, and/or each unit providing a dose of whey protein of 100 mg - 200 g.

The collagen peptide and/or whey protein as part of the combination as taught herein may be administered daily, once every other day, thrice weekly, twice weekly, or weekly. In addition or alternatively, the collagen peptide and/or whey protein as part of the combination as taught herein may be administered once a day, twice a day, or three times a day. In addition or alternatively, the collagen peptide and/or whey protein as part of the combination as taught herein may be administered for at least 1 day, or at 2 days, or at least 3 days, or at least 4 days, or at least 5 days, or at least 6 days, or at least 1 week, or at least 2 weeks, or at least 3 weeks, or at least 4 weeks, or at least 5 weeks, or at least 6 weeks, or at least 8 weeks, or at least 12 weeks, or at least 16 weeks.

In the various embodiments wherein the combination is administered to a subject performing exercise (e.g. exercise overload, strength training, and/or sports), the collagen peptide and/or whey protein as part of the combination is preferably administered starting 7 days, or 6 days, or 5 days, or 4 days, or 3 days, or 2 days, or 1 day before the start of the exercise, wherein the combination preferably may be administered twice daily, once daily, once every other day, thrice weekly, twice weekly, or weekly. In addition or alternatively, the combination is preferably administered for another 1 day, or 2 days, or 3 days, or 4 days, or 5 days, or 6 days, or 7 days after the end of the exercise, wherein the combination preferably may be administered twice daily, once daily, once every other day, thrice weekly, twice weekly, or weekly. In addition or alternatively, the combination is preferably administered 12 hours, 10 hours, 8 hours, 6 hours, 4 hours, 2 hours, 1 hour, 30 minutes, 15 minutes, 10 minutes, 5 minutes, and/or 1 minute before the start of the exercise. In addition or alternatively, the combination is preferably administered 1 minute, 5 minutes, 10 minutes, 15 minutes, 30 minutes, 1 hour, 2 hours, 4 hours, 8 hours, 10 hours, and/or 12 hours after the end of the exercise.

In various embodiments, the collagen peptide and whey protein as part of the current disclosure are administered simultaneously, separately, sequentially, and/or at intervals to a subject.

In an embodiment of the current disclosure, the unit comprising collagen peptide and the unit comprising whey protein are administered sequentially to a subject.

In an embodiment of the current disclosure, the daily amount of collagen peptide as taught herein and the daily amount of whey protein as taught herein are administered within a 24 hours timeframe, preferably within a 12 hours timeframe, most preferably within a 6 hours timeframe.

In embodiments of the current disclosure, the collagen peptide and whey protein are administered sequentially to a subject not more than 24 hours, or 12 hours, or 6 hours, or 4 hours, or 3 hours, or 2 hours, or 1 hour, or 30 min, or 15 min, or 10 min, or 5 min, or 1 min separated from each other, wherein the collagen peptide may be administered prior to the whey protein or vice versa.

In various embodiments, the collagen peptide and/or whey protein are provided in the combination (e.g. as part of the dosage forms as taught herein, the unit dose as taught herein and/or the composition as taught herein) in a food formulation, feed formulation, food supplement formulation, feed supplement formulation, or pharmaceutical formulation, preferably a food supplement formulation. In addition or alternatively, the collagen peptide and/or whey protein are preferably provided in the combination (e.g. as part of the dosage forms as taught herein, the unit dose as taught herein and/or the composition as taught herein) in a solid dosage form such as a capsule, a tablet, or a powder, preferably a powder.

In a preferred embodiment of the current invention, the collagen peptide and the whey protein are provided in a food formulation, feed formulation, food supplement formulation, feed supplement formulation, or pharmaceutical formulation, preferably a food supplement formulation.

In a preferred embodiment of the current invention, the collagen peptide and the whey protein are provided in a solid dosage form such as a capsule, a tablet, or a powder, preferably a powder.

In certain embodiments, the collagen peptide and/or whey protein as part of the combination as taught herein may be provided in a formulation such as a drinkable solution or suspension, drink, syrup, artificially-flavoured drink, carbonated beverage, (water-soluble) powdered mixture, (water-soluble) paste, (water-soluble) powder, (water-soluble) tablet, (water-soluble) pill, (water-soluble) dragee, (water-soluble) caplet, (water-soluble) sachet, or (water-soluble) capsule. In addition or alternatively, the collagen peptide and/or whey protein as part of the combination as taught herein may be present in a functional food, e.g. a juice, shake, dairy drink, yoghurt, yoghurt drink, dessert, energy bar, nutritional bar, slimming bar, or confectionery.

In a preferred embodiment, the current disclosure relates to a combination for use in decreasing inflammation in skeletal muscle inflammation, wherein the combination comprises collagen peptide and whey protein in a ratio of (CP:WP) 1:1 and 1:2 (the ratio based on the total dry weight amount administered to a subject per day and/or the dry weight amount per unit dose), wherein the collagen peptide and whey protein are provided as separate food formulations in the form of a powder.

In a preferred embodiment, the present disclosure describes a combination for use in treating tissue inflammation as taught herein, wherein the combination comprises collagen peptide and whey protein in a ratio of (CP:WP) 1:1 and 1:2 (the ratio based on the total dry weight amount administered to a subject per day and/or the dry weight amount per unit dose), wherein the collagen peptide and whey protein are provided as a single food formulation in the form of a powder.

In a preferred embodiment, the present disclosure describes a combination for use in treating a tissue inflammation-related condition as taught herein, wherein the combination comprises collagen peptide and whey protein in a ratio of (CP:WP) 1:1 and 1:2 (the ratio based on the total dry weight amount administered to a subject per day and/or the dry weight amount per unit dose), wherein the collagen peptide and whey protein are provided as separate food formulations in the form of a powder.

In a preferred embodiment, the present disclosure describes a combination for use in treating a tissue inflammation-related condition as taught herein, wherein the combination comprises collagen peptide and whey protein in a ratio of (CP:WP) 1:1 and 1:2 (the ratio based on the total dry weight amount administered to a subject per day and/or the dry weight amount per unit dose), wherein the collagen peptide and whey protein are provided as a single food formulation in the form of a powder.

In a preferred embodiment, the present disclosure describes a combination for use in treating a symptom of a tissue inflammation-related condition as taught herein, wherein the combination comprises collagen peptide and whey protein in a ratio of (CP:WP) 1:1 and 1:2 (the ratio based on the total dry weight amount administered to a subject per day and/or the dry weight amount per unit dose), wherein the collagen peptide and whey protein are provided as separate food formulations in the form of a powder.

In a preferred embodiment, the present disclosure describes a combination for use in treating a symptom of a tissue inflammation-related condition as taught herein, wherein the combination comprises collagen peptide and whey protein in a ratio of (CP:WP) 1:1 and 1:2 (the ratio based on the total dry weight amount administered to a subject per day and/or the dry weight amount per unit dose), wherein the collagen peptide and whey protein are provided as a single food formulation in the form of a powder.

In a preferred embodiment, the present disclosure describes a combination for use in treating a symptom of tissue inflammation as taught herein, wherein the combination comprises collagen peptide and whey protein in a ratio of (CP:WP) 1:1 and 1:2 (the ratio based on the total dry weight amount administered to a subject per day and/or the dry weight amount per unit dose), wherein the collagen peptide and whey protein are provided as separate food formulations in the form of a powder.

In a preferred embodiment, the present disclosure describes a combination for use in treating a symptom of tissue inflammation as taught herein, wherein the combination comprises collagen peptide and whey protein in a ratio of (CP:WP) 1:1 and 1:2 (the ratio based on the total dry weight amount administered to a subject per day and/or the dry weight amount per unit dose), wherein the collagen peptide and whey protein are provided as a single food formulation in the form of a powder.

In one aspect, the present disclosure describes a non-therapeutic and/or non-medical use of the combination as taught herein. For example, according to the present disclosure the combination as taught herein may be used in (one or more forms of) exercise as taught herein (e.g. wherein the exercise is exercise overload, strength training, and/or sports). The non-therapeutic and/or non-medical use as disclosed herein preferably relates to a use of the combination in the absence of inflammation, a tissue inflammation-related condition, and/or a symptom of tissue inflammation and/or a tissue inflammation-related condition. In addition or alternatively, the non-therapeutic and/or non-medical use as disclosed herein preferably relates to a use of the combination in the absence of a pathology or a sign thereof.

In one aspect, the current disclosure relates to a method of treating subjects experiencing tissue inflammation, a tissue inflammation-related condition, and/or a symptom of tissue inflammation and/or a tissue inflammation-related condition using the combination and/or composition comprising collagen peptide and whey protein as taught herein. The method of treatment may use any of the embodiments of the combination and/or composition comprising collagen peptide and whey protein as taught herein, including the disclosed dosages (e.g. unit doses or daily amounts), dosage forms, formulations, and administration methods. The tissue inflammation, tissue inflammation-related condition, and/or a symptom of tissue inflammation and/or a tissue inflammation-related condition may be any condition for which the combination and/or composition is suitable as taught herein. In a preferred embodiment, the method of treatment is preferably used to treat inflammation in exercise overload, strength training, and/or sports. In another preferred embodiment, the method of treatment is preferably used to treat inflammation in exercise-induced muscle damage, delayed onset muscle soreness, inflammatory myopathy, tendinopathy, tendinitis, arthritis, polychondritis, osteomyelitis, ankylosing spondylitis, inflammation-mediated bone loss, and/or autoinflammatory bone disease.

In one aspect, the disclosure relates to the use of the combination comprising collagen peptide and whey protein as taught herein for the manufacture of a medicament for the amelioration, prevention, and/or treatment of tissue inflammation, a tissue inflammation-related condition, and/or a symptom of tissue inflammation and/or a tissue inflammation-related condition. The combination and/or composition comprising collagen peptide and whey protein may use any of the embodiments of the combination and/or composition comprising collagen peptide and whey protein as taught herein, including the disclosed dosages (e.g. unit doses or daily amounts), dosage forms, formulations, and administration methods. The tissue inflammation, tissue inflammation-related condition, and/or a symptom of tissue inflammation and/or a tissue inflammation-related condition may be any condition for which the combination and/or composition is suitable as taught herein. In a preferred embodiment, the combination and/or composition comprising collagen peptide and whey protein is used for the manufacture of a medicament for the treatment of inflammation in exercise overload, strength training, and/or sports. In another preferred embodiment, the combination and/or composition comprising collagen peptide and whey protein is used for the manufacture of a medicament for the treatment of inflammation in exercise-induced muscle damage, delayed onset muscle soreness, inflammatory myopathy, tendinopathy, tendinitis, arthritis, polychondritis, osteomyelitis, ankylosing spondylitis, inflammation-mediated bone loss, and/or autoinflammatory bone disease.

### Brief Description of the Figures

- FIG. 1 is a chart showing changes in mouse body weight during the test period.
- FIG. 2A is a graph showing the wet weight of excised gastrocnemius muscle
- FIG. 2B is a graph showing the protein concentration in homogenized supernatant of the excised gastrocnemius muscle in a standardized amount per wet weight (specific weight).
- FIG. 3 is a graph showing the results of DNA microarrays, and is a graph showing the results of DNA microarrays for 23 genes in which upregulation of CTX-inducible muscle damage-related genes is alleviated by administration of a test substance.
- FIG. 4 is a graph showing the results of DNA microarrays, and is a graph showing the results of DNA microarrays for 17 genes in which upregulation of CTX-inducible muscle damage-related genes is further enhanced by administration of a whey protein.
- FIG. 5 is a graph showing the results of DNA microarrays, and is a graph showing the day 1 results of inflammation-associated genes in which upregulation of CTX-inducible muscle damage-related genes is alleviated by administration of a test substance.
- FIG. 6 is a graph showing the results of the lactate dehydrogenase (LDH) levels in the serum on day 1.
- FIG. 7A is a graph showing the visual analogue scale (VAS) muscle pain perception at training session (TR) 1 and TR17 during drop jumps, wherein subjects have taken whey protein alone or the combination of whey protein and collagen peptide.
- FIG. 7B is a graph showing the VAS patellar tendon pain perception at training session TR1 and TR17 at palpation, wherein subjects have taken whey protein alone or the combination of whey protein and collagen peptide.
- FIG. 7C is a graph showing the patellar tendon thickness and neovascularization (Ohberg score) at TR1, TR17, and posttest, wherein subjects have taken whey protein alone or the combination of whey protein and collagen peptide.
- FIG. 7D is a graph showing the total creatine kinase (CK) in the blood at pretest, TR2, and posttest, wherein subjects have taken whey protein alone or the combination of whey protein and collagen peptide.
- FIG. 7E is a graph showing the total creatine procollagen type-1 polypeptidase (P1NP) in the blood at pretest, TR2 and posttest, wherein subjects have taken whey protein alone or the combination of whey protein and collagen peptide.

### Experimental examples

Hereinafter, the present disclosure is described in more detail with reference to Experimental examples.

### Experimental example 1

### 1. Introduction

This study investigates the effect of collagen peptide and whey protein, alone or in combination, on alleviating muscle inflammation and damage using a mouse model of cardiotoxin (CTX)-induced muscle inflammation.

### 2. Methods

### 2.1. Test materials

- Collagen peptide: "Peptan P 5000HD" (trade name, manufactured by Rousselot), derived from pig skin, average molecular weight 5000, high specific gravity type (specific gravity: 0.40 to 0.55/cm³) (collagen peptide purity: 75-95%)
- Whey protein: "LACTOMIN80-S" (trade name, manufactured by Lactoprot Deutschland GmbH), 80% whey protein concentrate
- Muscle damage inducer, cardiotoxin: 15 µM manufactured by Latoxan

### 2.2. Animals and breeding environment

Mice (C57BL/6JJmsSlc, SPF, male): 7-week-old mice were received and bred under the following breeding environment for one week, and then divided into 5 groups of 5 mice each so that their body weights were evenly distributed. The breeding environment during the test period was the same as during the training period.

The breeding environment was as follows:
- Temperature: 18 to 28°C
- Relative humidity: 30 to 80%
- Lighting time: 12 hours (7:00 to 19:00)
- Feed: solid feed MF (manufactured by Oriental Yeast Co., Ltd.), free intake
- Drinking water: free intake

### 2.3. Animal test

A CTX-induced muscle damage model was prepared by intramuscularly administering Cardiotoxin (hereinafter, sometimes referred to as "CTX") to the middle abdomen of the gastrocnemius muscle of mice. At that time, collagen peptide, whey protein, or collagen peptide and whey protein were orally administered as test substances for 21 days using a sonde from 7 days before the CTX treatment to 14 days after the treatment.

Table 1 summarizes the test substance, dose, volume, CTX treatment status, CTX treatment volume, and number of cases for each test group.

**Table 1**

| Test group | Test substance | Dose (g/kg) | Volume (mg/mL) | CTX treatment status | CTX treatment volume (µL) | Number of cases |
|---|---|---|---|---|---|---|
| A1 | vehicle group (water for injection) | - | 10 | no | - | 5 |
| A2 | vehicle group (water for injection) | - | 10 | yes | 100 | 5 |
| A3 | collagen peptide | 2 | 10 | yes | 100 | 5 |
| A4 | whey protein | 4 | 10 | yes | 100 | 5 |
| A5 | collagen peptide + whey protein | 1 + 2 | 10 | yes | 100 | 5 |

On the day following the final day of administration, the mice were anesthetized, blood was collected from the abdominal aorta, exsanguinated, and then the gastrocnemius muscle was excised. A portion was removed for protein content determination and the wet weight was measured, and the remainder was immersed in a solution of an RNA stabilizer (trade name "RNAlater", manufactured by QIAGEN) for DNA microarray, left overnight in a refrigerator, removed from the solution and cryopreserved at -80°C or less.

### 2.4. Measurement of protein amount

Gastrocnemius muscle whose wet weight was measured was placed in a tube for a bead-type sample crusher, zirconium beads and a predetermined amount (1 mL) of phosphate buffered saline (PBS) were added, and the mixture was homogenized in the bead-type sample crusher for 3 minutes under cooling with dry ice. Centrifugation was carried out at 12,000 ×g at 40°C for 15 minutes, the supernatant was separated, and the protein concentration was measured using a measurement kit "DC Protein Assay Kit" (trade name, manufactured by BIO-RAD). According to the microplate protocol of the assay kit, the standard at that time was a PBS solubilized dilution series of BSA (Bovine Serum Albumin) ranging from 0.078 mg to 5 mg/mL. The absorbance at 630 nm was measured using a microplate reader "iMark" (trade name, manufactured by BIO-RAD).

From the BSA standard curve, the BSA-equivalent protein concentration of the sample was calculated. The results were standardized per wet weight, and each measured value was expressed as mean ± standard error. In addition, the significant difference was tested by the following statistical method.

### 2.5. Statistical method

A two-group test was performed on groups A1 and A2. The Student's t-test was performed when the F-test showed uniformity of variance, and the Aspin-Welch's t-test was performed when the F-test showed no uniformity of variance. In addition, multiple comparison tests were performed for each of the A2 to A5 groups. The multiple comparison test was parametric Dunnett's test when the Bartlett's test showed uniformity of variance, and non-parametric Steel's test when the Bartlett's test showed no uniformity of variance. The significance level was less than 5%.

### 2.6. DNA Microarray

The cryopreserved gastrocnemius muscle samples were quickly thawed, and each individual was subjected to nucleic acid extraction using "RNAiso plus" (trade name, manufactured by Takara Bio Inc.), followed by DNase treatment in the liquid phase to purify total RNA using a purification kit (trade name "RNeasy MinElute Cleanup Kit", manufactured by QIAGEN).

The quality control of the obtained total RNA was based on the RNA purity of A260/230 ratio of 1.5 or more and A260/280 ratio of 1.8 or more. Further, the resolution RIN (RNA Integrity Number) (trade name, manufactured by Agilent Technologies) was derived from the electrophoretic image of RNA, and only RNA having a RIN value of 7.0 or more out of 10 was used. Criteria conforming RNA was stored frozen (-70°C or below) until the next step.

RNA samples were pooled in each test group, and Cy3-labeled cRNA was synthesized and purified using a Cy3-labeled cRNA synthesis kit "Low Input Quick Amp Labeling Kit" (trade name, manufactured by Agilent Technologies). The concentration of the obtained labeled cDNA and the Cy3-CTP introduction rate were calculated from the absorbances at 260 nm, 280 nm, 550 nm, and 320 nm to confirm that the reference values (Cy3-CTP introduction rate > 6 pmol/µg) were satisfied.

The labeled cRNAs from each test group were fragmented using a microarray hybridization kit "Gene Expression Hybridization Kit" (trade name, manufactured by Agilent Technologies), submitted to an array for mouse gene expression analysis "Whole Mouse Genome Microarray Ver. 2.0" (trade name, manufactured by Agilent Technologies), and hybridized at 65°C for 17 hours. After that, the array slides were washed with the array cleaning solution "Gene Expression Wash Buffer" (trade name, manufactured by Agilent Technologies), and the array images scanned by the microarray scanner are quantified by the array analysis software "GenePix Pro" (trade name, manufactured by Molecular Devices). The fluorescence intensity values were standardized, and the ratio of the fluorescence intensity of group A1 (vehicle group; no administration of CTX) to that of group A2 (vehicle group; with administration of CTX), group A3 (collagen peptide single administration group), group A4 (whey protein single administration group), or group A5 (combined administration group with collagen peptide and whey protein) was calculated.

### 3. Results

### 3.1. Body weight

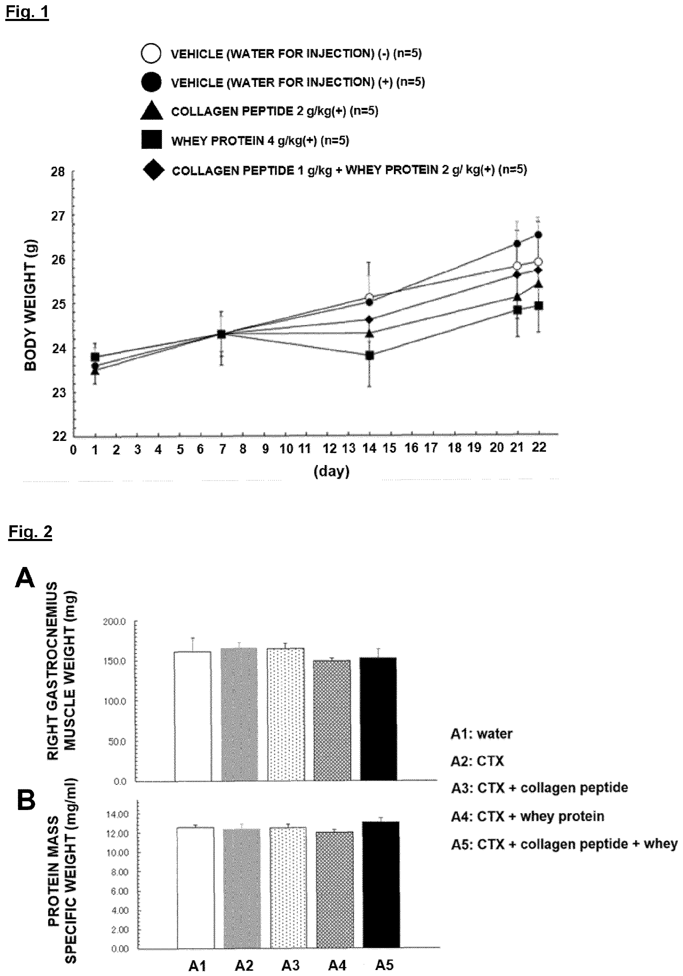

FIG. 1 shows changes in mouse body weight during the test period. As shown in FIG. 1, all of the test groups showed a steady increase in body weight throughout the administration period, and no significant difference was observed between the test groups. From this, it was shown that the administered substances were safe in both quantity and quality.

### 3.2. Gastrocnemius muscle weight and protein amount

FIGS. 2A and 2B show the wet weight of the excised gastrocnemius muscle and the protein concentration in the homogenized supernatant of the excised gastrocnemius muscle in a standardized amount per wet weight (specific weight).

As shown in FIG. 2A, the wet weight of the excised gastrocnemius muscle was 161.5 mg in group A1 (vehicle group; no administration of CTX), 165.5 mg in group A2 (vehicle group; with administration of CTX), 165.0 mg in group A3 (collagen peptide single administration group), 148.9 mg in group A4 (whey protein single administration group), and 153.2 mg in group A5 (combined administration group with collagen peptide and whey protein). No significant difference was found between the test groups in the significance test with a significance level of less than 5%.

As shown in FIG. 2B, the protein amount (specific weight) of the excised gastrocnemius muscle was 12.56 mg/mL in group A1 (vehicle group; no administration of CTX), 12.38 mg in group A2 (vehicle group; with administration of CTX), 12.50 mg/mL in group A3 (collagen peptide single administration group), 11.99 mg/mL in group A4 (whey protein single administration group), and 13.07 mg/mL in group A5 (combined administration group with collagen peptide and whey protein). No significant difference was found between the test groups in the significance test with a significance level of less than 5%. These results indicate that this muscle damage model does not cause major damage to the entire muscle and is a suitable test system from the viewpoint of microdamage.

### 3.3. DNA Microarray

### 3.3.1. Alleviation of upregulation of CTX-inducible muscle damage-related genes, Part 1

In the medium-administered group to which the muscle damage inducer CTX was administered, 414 genes showed upregulation as compared with the medium-administered group to which CTX was not administered. Of these upregulated genes, the genes whose 2-fold upregulation was alleviated to less than 1.14 fold by the administration of the test substance were extracted, and the following 23 genes were extracted:
- Spp1: secreted phosphoprotein 1
- Igtax: integrin alpha X
- Gpnmb: glycoprotein (transmembrane) nmb
- Clec7a: C-type lectin domain family 7, member a
- Trem2: triggering receptor expressed on myeloid cells 2
- Lgals3: galectin-3
- C1qb: complement compoaxnent 1, q subcomponent, beta polyeptide
- Ctss: cathepsin S
- H2-Aa: histocompatibility 2, class II antigen A, alpha
- Cd83: CD83 antigen
- Isg15: ISG15 ubiquitin-like modifier
- Oasl1: 2'-5' Oligoadenylate synthetase-like 1
- Laptm5: lysosomal-associated protein transmembrane 5
- Gm7325: predicted gene 7325
- Krt18: keratin 18
- Igf2: insulin-like growth factor 2
- Myog: myogenin
- Tpm3: tropomyosin 3, gama
- Ablim1: actin-binding LIM protein 1
- Clec3b: C-type lectin domain family 3, member b
- Mest: mesoderm specific transcript
- Kcne1L: potassium voltage-gated channel, Isk-related family, member L-like
- BC028528: cDNA sequence BC028528

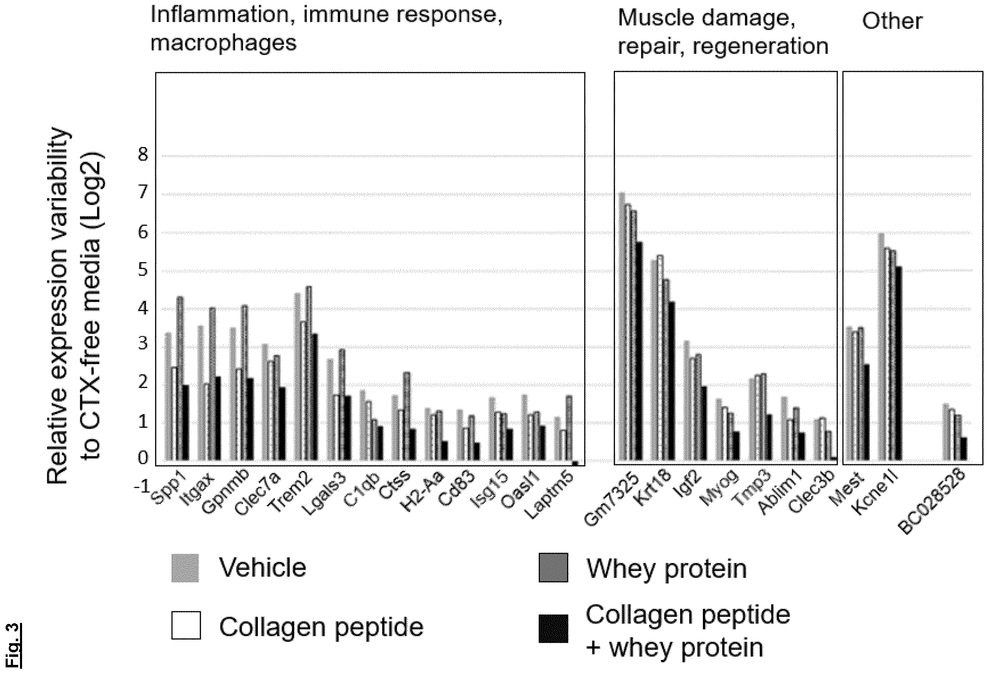

The results are shown in Table 2 and FIG 3.

**Table 2**

| | Test group | Test substance | Number of genes | Gene name |
|---|---|---|---|---|
| Alleviate upregulation | A3 | collagen peptide | 4 | genes involved in inflammatory response and macrophages Spp1, Itgax, Gpnmb, Lgals3 |
| | A4 | whey protein | 0 | |
| | A5 | collagen peptide + whey protein | 23 | Genes involved in inflammatory and immune responses Spp1, Igtax, Gpnmb, Clec7a, Trem2, Lgals3, C1qb, Ctss, H2-Aa, Cd83, Isg15, Oasl1, Laptm5 |
| | | | | Genes with numerous reports on muscle damage, repair, regeneration, and differentiation Gm7325, Krt18, Igf2, Myog, Tpm3, Ablim1, Clec3b, Mest, Kcne1 |
| | | | | Others BC028528 |

As shown in Table 2 and FIG. 3, the number of genes that alleviated the upregulation by administration of CTX, which is a muscle damage inducer, was 4 in the collagen peptide single administration group (group A3), and the whey protein single administration group. The number was 0 in (group A4), but 23 in the test group (group A5) in which collagen peptide and whey protein were administered in combination.

### 3.3.2. Alleviation of upregulation of CTX-inducible muscle damage-related genes, Part 2

Out of the 414 genes whose expression was upregulated in the medium-administered group to which the muscle damage inducer CTX was administered, the upregulation of the following 17 genes was further enhanced in the whey protein single administration group (group A4). On the other hand, in the collagen peptide single administration group (group A3) and the test group in which the collagen peptide and whey protein were administered in combination (A5 group), such an increase in expression was not observed. The following markers are of interest:
- Lst1: leukocyte specific transcript 1
- Emr1: EGF-like containing, mucin-like, hormone receptor-like sequence 1
- Aif1: allograft inflammatory factor 1
- Clec4a1: C-type lectin domain family 4, member a1
- Slamf9: SLAM family member 9
- Fcer1g: Fc receptor, IgE, high affinity 1, gamma polypeptide
- Lcp1: lymphocyte cytosolic protein 1
- Unc93b1: unc-93 homlog B1
- Cd14: CD14 antigen
   Ly86: lymphocyte antigen 86
- Lilrb3: leukocyte immunoglobulin-like receptor, subfamily B (with TM and ITIM domains), member3
- Sirp a: signal-regulatory protein alpha
- Mpeg1: macrophage expressed gene 1
- Spp1: secreted phosphoprotein 1
- Tyrobp: TYRO protein tyrosine kinase binding protein
- Cyth4: cytohesin 4
- Slc11a1: solute carrier family 11 (proton-coupled divalent metalion transporters), member1

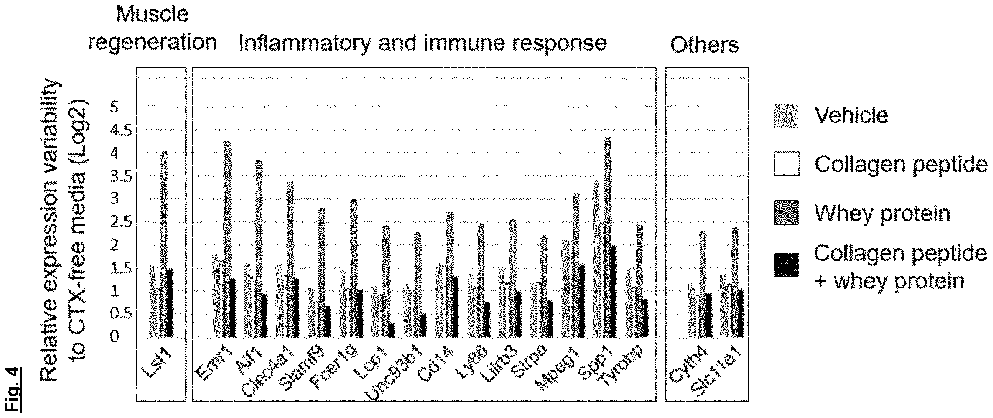

The results are shown in Table 3 and FIG 4.

**Table 3**

| | Test group | Test substance | Number of genes | Gene name |
|---|---|---|---|---|
| Further enhance upregulation | A3 | collagen peptide | 0 | |
| | A4 | whey protein | 17 | Genes involved in muscle damage, repair, regeneration Lst1 |
| | | | | Genes involved in inflammatory and immune responses Emr1, Aif1, Clec4a1, Slamf9, Fcer1g, Lcp1, Unc93b1, Cd14, Ly86, Lilrb3, Sirpa, Mpeg1, Spp1, Tyrobp Others Cyth4, Slc11a1 |
| | A5 | collagen peptide + whey protein | 0 | |

As shown in Table 3 and FIG. 4, the further upregulation of the CTX-inducible gene by administration of whey protein was alleviated to the level of vehicle administration or less by the combined use with CP.

### 4. Test evaluation

As a result of the DNA microarray, it was considered that the effect that did not occur by the single administration of collagen peptide or whey protein, but only in the combined administration group.

The phenomenon of alleviating the upregulation of CTX-inducible genes can be regarded as the result of accelerated repair of muscle damage.

Specifically, in the collagen peptide single administration group, the upregulation of the CTX-induced gene was alleviated in the gene involved in the inflammatory response, but since collagen peptide has been reported to be effective in suppressing inflammatory responses associated with osteoarthritis of the knee, the results of the collagen peptide single administration group were considered reasonable.

On the other hand, in the combined administration group of collagen peptide and whey protein, a suppressive effect was also observed in genes involved in inflammatory responses observed in the collagen peptide single administration group, and no changes were observed in the collagen peptide or whey protein single administration group. In addition, the relaxation of CTX-inducible genes was observed in a group of genes reported to be involved in damage, repair, regeneration, and differentiation, suggesting an effect on muscle damage recovery. Moreover, the effect in the combined administration group was remarkable at a combined dose of half the dose of the test substance in the single administration group.

In general, muscle damage recovery involves the following steps:
- First stage: Infiltration of inflammatory cells such as neutrophils and macrophages occurs immediately after damage. At the same time, myosatellite cells are activated by cytokines and growth factors released from these cells.
- Second stage: Between a few hours and a few days after damage, activated myosatellite cells proliferate and differentiate into myoblasts.
- Third stage: In parallel, myoblasts fuse with each other over a period of about one week to form multinucleated myotubular cells.
- Fourth stage: Myotubular cells become hypertrophied and transformed into mature muscle fibers.

Based on the above, the current inventors have found that the combination of collagen peptide and whey protein inhibits the upregulation of a large array of CTX-induced genes involved in muscle damage, repair, regeneration, cell differentiation and/or the immune responses. Particularly surprising, the upregulation of genes associated with tissue inflammation was only alleviated by the combination of collagen peptide and whey protein. This indicates that there is a synergistic interaction between collagen peptide and whey protein in the regulation of tissue inflammation, such as decreasing excessive and/or catabolic tissue inflammation.

The alleviation of tissue inflammation may involve one or both innate and adaptive immune pathways. To illustrate, on the one hand, CD11c is a type I transmembrane protein found at high levels on most human dendritic cells, but also on monocytes, macrophages, and neutrophils, which have key roles in the innate immune response. On the other hand, SSP1 is the gene for the osteopontin protein, which functions as a Th1 cytokine, promotes cell-mediated immune responses, and plays a role in chronic inflammatory and autoimmune diseases.

The alleviation of tissue inflammation is found to be associated with a decreased expression of genes involved in muscle damage. For example, the decrease in genes associated with inflammation was coupled to a decrease in the KRT18 and Clec3B genes, which encodes for important effectors of tissue and muscle scarring. At the same time, the inventors found that the alleviated expression of the genes Gm7325, Myog, Tpm3, Ablim1, and Mest indicates a (partial) reversal of an abnormal remodelling of the muscle tissue caused by CTX.

In addition, the current inventors found that the data unexpectedly show that the administration of whey protein may further promote CTX-induced genes involved in muscle damage, repair, cell differentiation, and/or the inflammatory/immune response. This (unfavourable) effect of whey protein however seems counteracted when it is co-administered with collagen peptide.

The exaggeration of tissue inflammation by whey protein may involve one or both innate and adaptive immune pathways. To illustrate, on the one hand, the Emr1CD11c, Ai41 and CD14 genes encode for proteins that are markers of monocytes/macrophages. On the other hand, Lcp-1 and SSP1 are markers linked to adaptive immune responses, in particular T lymphocytes.

The results of this study showed that the ingestion of collagen peptide and whey protein in combination was effective in muscle damage recovery, as it promoted early convergence of inflammatory reactions and muscle repair, regeneration, and differentiation during muscle damage.

### 5. Preparation example

The following is a prescription example constituting the combination for promoting muscle damage recovery.

### 5.1. Prescription example 1: Powdered beverage composition

- Whey protein WWP8010 (trade name, Nippon Shinyaku) 65% by mass
- Collagen peptide Peptan P 5000HD (trade name, Rousselot) 20% by mass
- Granulated sugar 7% by mass
- Cocoa powder 7% by mass
- Sucralose 0.4% by mass
- Chocolate flavour 0.3% by mass
- Emulsifier 0.3% by mass

About 20 g of this powdered beverage composition was weighed and dissolved in 200 to 300 mL of water, soymilk, milk or the like to prepare a beverage. The obtained beverage was a beverage for daily intake as a beverage for consumption before and after exercise, snack, and beverage for consumption before going to bed, with an intake of 50 to 600 mL/day.

### 5.2. Prescription example 2: Bar confectionery

- Soy puff 43.3% by mass
- Granulated sugar 14.4% by mass
- Shortening 13.1% by mass
- Collagen peptide Peptan P 5000HD (trade name, Rousselot) 13.1% by mass
- Whey protein WWP9010 (trade name, Nippon Shinyaku) 8% by mass
- Cocoa powder 3% by mass
- Almond 3% by mass
- Cocoa mass 1% by mass
- Chocolate flavour 0.7% by mass
- Emulsifier 0.3% by mass
- Sucralose 0.1% by mass

This bar confectionery was a bar confectionery that can be ingested on a daily basis at 25 to 60 g/day as a substitute food for breakfast, food for ingestion before and after exercise, and snacks.

### 5.3. Prescription example 3: Drink

- Granulated sugar 15% by mass
- Collagen peptide Peptan P 5000HD (trade name, Rousselot) 3.2% by mass
- Whey protein lactocrystal plus (trade name, Nippon Shinyaku) 3.2% by mass
- Citric acid 0.7% by mass
- Agar 0.2% by mass
- Flavour 0.2% by mass
- Water 77.5% by mass
- This drink was a drink for daily intake with an intake of 50 to 200 mL/day as a drink for ingestion before and after exercise, a snack, or the like.

### Experimental example 2

### 1. Introduction

This study is a follow-up of the study as reported under Experimental example 1.

Experimental example 1 already demonstrated that the combined administration of collagen peptide and whey protein administration alleviates muscle inflammation and damage 15 days after CTX injection. In this example, the combined administration of collagen peptide and whey protein to alleviate the very early (acute) inflammation is also demonstrated.

### 2. Methods

This study investigates the effect of collagen peptide (CP) and whey protein (WP) administration, combined or alone, on alleviating muscle inflammation and damage after 1 day of CTX injection.

This study followed the same methodology as Experimental example 1. In addition or alternatively, in the current study:
- animals were euthanized 1 day after CTX treatment to perform the DNA microarray on muscle tissue
- an extra test group (group A6) was included, wherein animals received a double dose of collagen peptide and whey protein. This extra test group was added to clarify the range of intake
- a commercial lactate dehydrogenase (LDH) assay was used to determine the LDH serum levels as a measure of cell toxicity and tissue damage

Table 4 summarizes the test substance, dose, volume, CTX treatment status, CTX treatment volume, and number of cases for each test group.

**Table 4**

| Test group | Test substance | Dose (g/kg) | Volume (mg/mL) | CTX treatment status | CTX treatment volume (µL) | Number of cases |
|---|---|---|---|---|---|---|
| A1 | vehicle group (water for injection) | - | 10 | no | - | 3 |
| A2 | vehicle group (water for injection) | - | 10 | yes | 100 | 3 |
| A3 | collagen peptide | 2 | 10 | yes | 100 | 3 |
| A4 | whey protein | 4 | 10 | yes | 100 | 3 |
| A5 | collagen peptide + whey protein Half dose | 1 + 2 | 10 | yes | 100 | 3 |
| A6 | collagen peptide + whey protein Full dose | 2+4 | 10 | yes | 100 | 3 |

### 3. Results

### 3.1. DNA microarray

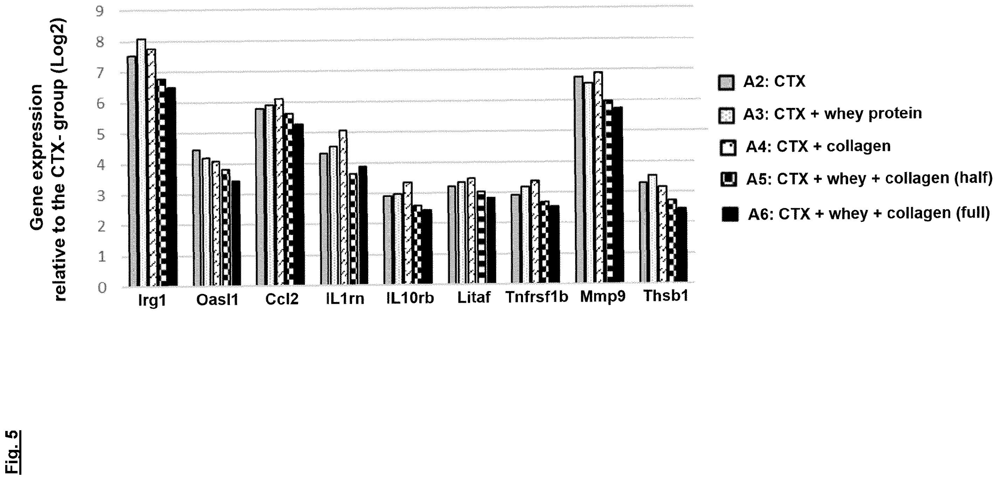

As shown in FIG. 5, combined administration of collagen peptide and whey protein (groups A5, A6) synergistically alleviated the expression of several genes associated with tissue inflammation, i.e. Irg1, Oasl1, Ccl2, IL1rn, IL-10rb, Litaf, Tnfrsf1bm and Mmp9. No differences were found between the combination groups receiving the half (group A5) or full dose (group A6) of collagen peptide and whey protein. An alleviation of the genes was not seen after the single administration of whey protein (group 3) or collagen peptide (group 4)

### 3.2. LDH assay

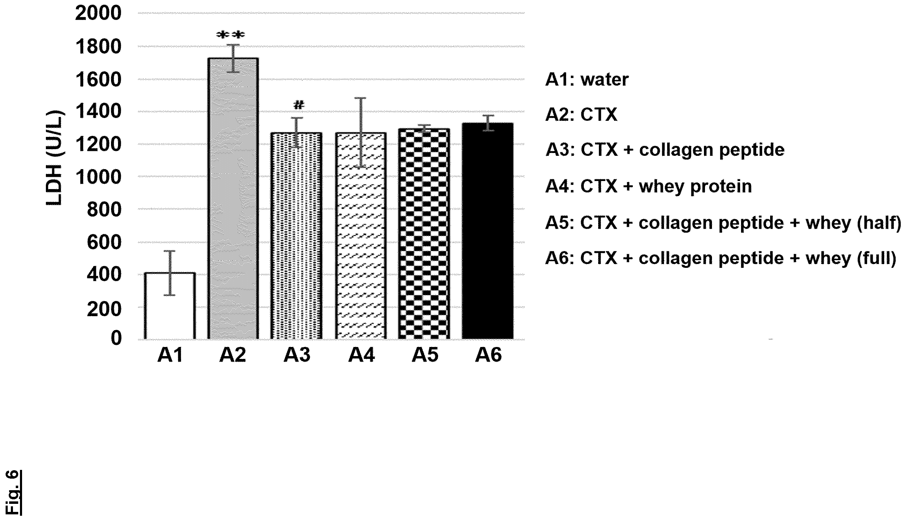

As shown in FIG. 6, an excessive increase in serum LDH was induced following CTX injection in the muscle. The LDH activity was suppressed in groups receiving collagen peptide and/or whey protein.

### 4. Test evaluation

The current DNA microarray data further strengthen the observations already made in Experimental example 1, namely that the combination of collagen peptide and whey protein inhibits the upregulation of genes associated with muscle damage and/or the immune response. Additionally, the current findings shed new light on the mechanism by which the combination of collagen peptide and whey protein may modulate inflammation and have protective effects on the muscle tissue.

Already on day 1, the following markers were found to be alleviated by the combination of collagen protein and whey protein :
- Irg1: encodes for a protein that is highly expressed in M1 macrophages (inflammation-inducing macrophages) and produces itaconic acid from cis-aconitic acid in the citric acid cycle. Itaconic acid acts on M1 itself to induce changes to M2 macrophages (anti-inflammatory macrophages). It suggests that M2 induction was performed earlier than the control group.
- Oasl1: Expression is increased by type I interferon, and OASL1 protein suppresses interferon production (negative feedback).
- Ccl2: a chemokine also referred to as monocyte chemoattractant protein. It recruits monocytes/macrophages, memory T cells, and dendritic cells to sites of tissue inflammation.
- IL1rn: gene that encodes for the Interleukin-1 receptor antagonist protein (IL1Ra). IL1Ra is secreted by various types of cells including immune cells, epithelial cells, and adipocytes, and is a natural inhibitor of the pro-inflammatory effect of IL1β. This protein inhibits the activities of interleukin 1, alpha (IL1A) and interleukin 1, beta (IL1B), and modulates a variety of interleukin 1 related immune and inflammatory responses.
- IL10rb: the protein encoded by this gene belongs to the cytokine receptor family. It is an accessory chain essential for the active interleukin 10 receptor complex and is upregulated in the inflammatory response.
- Litaf: encodes for the protein Lipopolysaccharide-induced tumor necrosis factor-alpha factor, which regulates the expression of numerous inflammation-associated cytokines, such as TNF, CCL2, CCL5, CXCL1, IL1A and IL1.
- Tnfrsf1b: the protein encoded by this gene is a member of the tumor necrosis factor receptor (TNF) superfamily and is demonstrated to be an important mediator of TNF function.
- Mmp9: Mmp9 is one of the secretory metalloproteases, involved in the degradation of the extracellular matrix. Decreased Mmp9 gene expression is indicative of decreased ECM degradation or tissue damage.
- Thbs1: The encoded thrombospondin has an angiogenesis-inhibiting effect. A decreased thbs1 gene expression is indicative of an enhanced angiogenic response associated with tissue repair and an anti-inflammatory response.

Together, the downregulation of these genes points towards a damped inflammatory response (decreases expression of inflammation associated cytokines), which was furthermore linked to a pro-regenerative milieu (induction of M2 macrophages, enhanced angiogenic response). Since this effect was seen already 1 day after CTX administration, it is considered that the inflammation of the muscles converged early in the combination group and promptly shifted to the repair process.

In addition, the comparison of two doses of collagen peptide and whey protein did not show a difference in efficacy between the two, therefore an optimal amount of intake has been established.

Finally, the LDH data furthermore supports that the combination of collagen peptide and whey protein may additionally have protective effects on cell toxicity and tissue damage caused by inflammation.

### Experimental example 3

### 1. Introduction

The following sections describe a clinical study investigating the short-term and acute effects of the combination of collagen peptide and whey protein on:
1. Changes in muscular functional capacity during exercise exercise overload.
2. Muscle and tendon damage and repair during exercise exercise overload.
3. Local and systemic inflammation.
4. Pain perception (muscle, tendon, general), including delayed-onset-of-muscle-soreness (DOMS), during exercise exercise overload.

### 2. Methods

### 2.1. Subject recruitment

Healthy male subjects (n = 40) are recruited for voluntary participation in this study. The sample sizes are calculated based on the expected effect size (see power analysis below) and an anticipated drop-out rate of 10-15%. Participants are recruited from a student population, by invitation via Email, lectures, posters, and social media.

In phase 1, candidate subjects are screened on compatibility with the inclusion and exclusion criteria by one of the investigators.

Inclusion criteria:
- Males between 18 and 30 years old.
- Sports participation, including general fitness training, between 2 and 5 hours max per week
- Body mass index between 18.5 and 25

Exclusion criteria:
- Any kind of injury/pathology that is a contra-indication to perform high-intensity exercise
- Intake of any medication or nutritional supplement that could impact muscle protein synthesis and/or exercise performance during the period of the study
- Intake of any whey protein, casein of BCAA supplement from 1 month prior to the start of the study
- Blood donation within 3 months prior to the start of the study
- Smoking
- Current participation in another research trial
- Any other argument to believe that the subject is unlikely to successfully complete the full study protocol

In phase 2, initially 40 'priority' subjects are invited for a standard medical screening by a certified sports physician. The screening includes a standard medical questionnaire and anamnesis, a resting electrocardiogram, and measurements of blood pressure and body weight. If subjects are excluded for participation due to the medical screening, additional subjects are medically screened to eventually obtain 40 subjects meeting all inclusion/exclusion criteria. Based on long-term experience, we anticipate that from the 40 subjects initially undergoing the medical screening following priori selection based on the above inclusion/exclusion criteria, <5% of the subjects are needed to be replaced by other candidates.

### 2.2. Study protocol

### 2.2.1. Study design

A randomized double-blinded placebo-controlled study is performed (n = 40). The timeline for the different measurements throughout the study is schematically presented in Table 4. The study involves a pretest (day -8) and a posttest (day 22), interspersed by a 1-week run-in period (day -7 to day -1) and a 3-week exercise training period (day 1 to day 20).

Furthermore, with the purpose to also allow evaluation of the acute effects of the training sessions on some specific endpoints (see Table 4) intermediate test sessions are organized on days 1 and 3, i.e. 1 hour and 48 hours after the first training session (TR1), respectively, as well as on day 20, i.e. 1 hour after the final training session.

During the run-in week and the training period the subjects receive either placebo (PL) or Collagen Peptan^{®} (CP) supplements as described below. The run-in phase serves to habituate the subjects to the dietary supplementation protocol, as well as to assure adequate protein availability in the direct approach of the experimental training period. The training program involves 6 weekly training sessions and specifically aims to induce transient muscular as well as patellar tendon overload, resulting in local inflammatory responses and tendinous pain within the 3-week time window.

Treatment allocation is done according to a matched-pairs randomization procedure. The subjects are pair-matched for body weight and countermovement jump-performance (primary output variable), after which the pairs are randomly split into the PL (n = 20) and CP group (n = 20). Nowhere during the study the test product and placebo are revealed, neither are identity concealment codes used in the randomization list. The randomization list is in the safe custody of the principal investigator only. The investigator does not record the identity of the revealed product in the rest of the participant's documents and does not disclose it to the subject or other study members.

### 2.2.2. Training program

The 3-week training program consists of 17 identical training sessions (TR1 to TR17). The training period starts on day 1 with TR1. Day 2 is a rest day, but from day 3 the subjects participate in a daily training session, except on Sundays.

The exercises chosen serve to deliver mainly eccentric contractions and thereby cause muscle damage in the knee-extensor muscles, while at the same time causing excess patellar tendon load. All training sessions are performed between 8 and 12 am, and always simultaneously for the matched pairs. Each session consists of three exercises: drop jumps (40 cm drop, 5 sets of 20 reps with 2 min rest between sets), knee-extensions on a knee-extension device (15RM workload, 5 sets of 20 reps with 2 min rest between sets), and eccentric knee-flexions (180° to 90° knee-angle) on a 30°downhill platform (5 sets of 20 reps with 2 min rest in between). A 5-min rest-interval intersperses the different exercises. All exercises are done unilaterally with the right leg only, using the left leg as non-exercised 'control leg'. The exercise prescriptions are identical for all training sessions (TR1 to TR17). However, whenever subjects are unable to fulfil the complete training program due to muscular fatigue or pain, the training load is reduced. But in order to allow quantification of the total work done over the full training period in each subject, the number of sets and reps performed per session, as well as the weight lifted in the knee-extension exercises, is noted in the individual training diary.

### 2.2.3. Collagen supplementation protocol

The supplements are delivered to the investigators in identical sachets containing either 15 grams or 30 grams of protein powder each. PL supplements contain either 15 g whey protein powder, or 30 g whey protein powder (6D Sports Nutrition - Whey Protein, vanilla flavour). CP supplements contain either 10 g Collagen Peptan^{®} powder mixed with 5 g whey protein powder, or 10 g Collagen Peptan^{®} powder added to 20 g of whey protein powder (6D Sports Nutrition - Whey Protein, vanilla flavour). PL and CP supplements are isocaloric (45 g total protein supplementation per day = 180 kcal per day) and are identical in appearance and flavour (vanilla).

The powders are provided in bulk by 6D Sports Nutrition (Antwerp, Belgium). The well-mixed experimental powders are then transferred into the experimental sachets by Rousselot (Gent, Belgium). Besides the double-blind identification and subject codes, each sachet also contains the instruction that the total volume of powder must be dissolved in 250-350 mL of water using a shaker (6D Sports Nutrition) that is provided by the investigators. The supplements are delivered to the investigators in two separate boxes per individual, one box containing the 15 g supplement sachets (n = 30), and one box containing the 30 g supplement sachets (n = 60). Sachets are identical in appearance for CP and PL to allowing blinded matched-pairs treatment allocation. However, 15 g and 30 g sachets must have a clearly different appearance so as to prevent dosage errors during the experiments.

The supplementation is started 1 week before the start of the training program (run-in period). During this run-in week (day -7 to day -1) the subjects ingest the 15 g protein supplement before breakfast, and the 30g protein supplement between 1 and 2 hours after breakfast. During the training period the subjects ingest the 15 g supplement 1 hour before the start of each training session, and the 30 g supplement immediately at the end of the session. On rest days (day 2, day 21 + Sundays) the subjects ingest the supplement similar to the run-in period, i.e. 15 g sachet before breakfast versus 30 g sachet between 1 and 2 hours after breakfast.

### 2.2.4. Protocols of experimental sessions - Pretest and posttest

The pretest (day -8) and the posttest (day 22) aim to evaluate the effects of short-term PL versus CP administration in conjunction with training on the primary and secondary endpoints, while avoiding the acute effects of the preceding training session. Hence the posttest is scheduled 48 hours after the last training session (TR17). The pretest and the posttest involve the full series of measurements (see below) aiming to assess (1) muscular functional capacity, (2) the degree of training-induced muscle and tendon tissue damage and repair, and (3) training-induced muscular and patellar tendon pain.

On both the pretest and the posttest the subjects report to the laboratory between 7 and 10 am. They receive a standardized light breakfast (~500 kcal, 65 E% carbohydrates, 20 E% protein, 15 E% fat). One hour later they start the test protocol which takes about two hours and involves (1) patellar tendon ultrasonography, (2) venous blood sampling, (3) a needle biopsies of the right and left m. vastus lateralis, and finally (4) the muscular functional capacity testing, in this order. The subjects receive 500 mL of water to drink during the entire test protocol which lasts about 2 hours. No other drinks or foods are allowed during the session, and no supplements are ingested on the days of the pretest and the posttest.

The pretest is scheduled on the day before the start of the run-in week (day -8), the posttest is done on day 22, i.e. 48 hours after the final training session (TR17) in order to avoid the acute effect of exercise on the parameters measured. Pretest and posttest protocols are identical and in every matched pair are done on the same day of the week and same time of the day.

### 2.2.5. Protocols of experimental sessions - Intermediate measurements

The intermediate test sessions serve to evaluate the acute effects of the training sessions either on tendon damage and repair, or on muscular damage and repair, or pain perception. On day 1 and day 20 patellar tendon ultrasonography is done 1h after the preceding training session. Therefore, at the end of TR1 and TR17, the subjects rest for 1 hour in a comfortable chair before undergoing the ultrasonography. Furthermore, on day 3 the subjects report to the laboratory in the morning in the fasted state and 1 hour before the start of the training session (TR2). They rest for 15 min in a comfortable chair before a blood sample is taken from an arm vein. The timing of this blood sample is explained by the fact that symptoms of muscle cell breakdown (~serum CK increase) and delayed onset of muscle soreness (DOMS) are most explicit by about 48 hours after an inaugural eccentric exercise training session.

### 2.3. Measurements

### 2.3.1. Muscular functional capacity

Muscular power is tested via countermovement jumps on a force platform (https://www.fusionsport.com/smartspeed-accessories/smartjump/). Maximal isometric knee extension force is assessed on an isokinetic dynamometer (https://www.biodex.com/physicalmedicine/products/dynamometers/system-4-pro). For each exercise the subjects receive 5 attempts with 1-min rest episodes in between, and the best of-5 is taken as the final output variable. Subjects perform a standardized 5-min warming-up before (1) the countermovement jumps and (2) the isometric knee extensions are performed in this order, and interspersed by a 5-min rest period.

### 2.3.2. Blood samples

Blood (2 x 10 mL) is sampled from an arm vein (Venoject^{®} system), and serum is immediately separated by centrifugation. Serum and plasma samples are distributed in different 1 mL aliquots and stored at -20°C until used for biochemical assays at a later date.

The plasma samples are analysed by appropriate specific ELISA assays for CK (marker for muscle damage), GDF15 (marker of overall physiological stress), P1NP, C1TP and P3NP (markers of collagen turnover) and endostatin, tumstatin and endorepellin (markers of extracellular matrix cleavage).

### 2.3.3. Ultrasonography

Ultrasonography is used to evaluate the effect of the overload training program on patellar tendon integrity. The ultrasound device used is MyLabSigma (Esaote^{®}) with a 5-16 MHz linear transducer. Measurements are performed bilaterally in order to be able to compare the trained (right) and the untrained (left) leg. Patellar tendon anteroposterior thickness in the proximal, middle, and distal region of the patellar tendon is measured by gray-scale ultrasonography (in millimeters). Patellar tendon neovascularization is measured by power color doppler ultrasonography and scored using the Modified Ohberg score (grade I = one vessel in tendon; grade II = two vessels in tendon; grade III = neovessels < 50% tendon thickness; grade IV = neovessels in 50-90% tendon thickness; grade V = neovessels > 90% tendon thickness).

### 2.3.4. Muscle biopsies

Muscle biopsies are taken from a subgroup of twelve volunteering subjects in each experimental group. Biopsies are taken from both the trained (right) and untrained (left) m. vastus lateralis by means of Bergström biopsy needles with suction being applied and under local anesthetic according to the procedures previously described (van Thienen et al. Physiol Rep. 2014 May 1; 2(5): e00286). Hence a total of 4 biopsies are taken per subject throughout the whole study, of which two in the left leg and two in the right leg. Biopsies are taken from m. vastus lateralis using a Bergström needle with a 3 mm diameter. First a small incision (~5mm) is made through the skin and underlying fascia under local anaesthesia (Lidocaïne, 1ml) to facilitate smooth penetration of the needle into the muscle belly. During both the pretest and the posttest, interspersed by the 3-week training period, one biopsy is taken from both XL and CL. After cutting of the tissue (~50-300 mg of muscle) the samples blood residues are rapidly washed of and connective tissue is removed before being frozen in liquid nitrogen and stored at -80°C until biochemical and histological analyses are done at a later date.

Samples are first analysed for integrin, desmin, and dystrophin content both at the protein level (Western blot) and at the mRNA level by real-time qPCR. Assays performed on the muscle samples target the metabolic pathways involved in regulation of muscle protein and collagen synthesis and breakdown.

### 2.3.5. Pain perception

Pain perception is assessed at different times during the study using both VAS scoring on a Wong-Baker rating scale, and the VISA-P questionnaire which has been well validated in to identifying tendinopathy in young active and otherwise healthy individuals (https://www.ncbi.nlm.nih.gov/pubmed/27374017). VAS rating of muscular pain is assessed immediately after the 40 cm drop-jump sets in TR1, TR2, TR8 and TR17. VISA-P questionnaire is applied before the ultrasonography sessions in the pretest and the posttest as well as in the intermediate test sessions on day 1 and day 20. Pain is assessed for both for the trained (right) and untrained (left) leg.

### 2.4. Measurement of inflammation-associated markers

The plasma samples and are analysed for systemic inflammation by appropriate specific ELISA assays for IL-6, IL-8, IL- β, IL-10, and TNF-α, IL-17 and CCL-2 (marker of and inflammation).

The biopsies are analysed for local tissue inflammation for an array of immune cell- and inflammation-associated markers at the mRNA level by real-time qPCR, including:
- CD86, CD163, CD11b, CD14, CD16 (macrophage markers)
- CD66b, CD11b, and myeloperoxidase (neutrophil markers)
- MHC class II, BDCA-1 (dendritic cell markers)
- CD3, CD4, CD8, CD25, CD19 (lymphocytes)
- Toll-like receptors (TLR, TLR2, TLR4, TLR7, TLR8, TLR9)
- IL-6, IL-8, IL- β, IL-10, and TNF-α, IL-17 and CCL-2 (inflammatory cytokines)

Inflammation in the tendon is determined (semi-)quantitatively using a scoring system for ultrasound based on reported methods (e.g. Gemignani et al. Emerg Radiol. 2008 Nov;15(6):399-404)

### 2.5. Dietary control and follow-up

The subjects receive standardized meals on the evening before (dinner) and the morning (breakfast) of the pretest and the posttest. Furthermore, the subjects complete a 3-day food diary during the run-in week (days -3 to -1) and during the final 3 days of the training period (days 18-20) in order to check for possible dietary variation between the groups. Dietary analyses are performed using a web-platform (https://mijn.voedingscentrum.nl/nl/eetmeter/) for dietary recording and analysis of macronutrient and micronutrient composition.

### 2.6. Statistical analyses

Statistical analyses is performed using appropriate parametric methods using Statistica software. This includes unpaired t-testing and 2-way analyses of variance for repeated measures where appropriate using Holm-Sidak's multiple comparison testing for post hoc analyses whenever appropriate. All statistical significances are considered at the p<0.05 level, or 95% confidence interval. The statistical analyses are carried out by a member of the study team who is fully blinded to the subject group allocation.

An a priori power analysis (G*Power 3.1) indicates that a significant effect at 5% probability level evaluated by a two-way repeated measures ANOVA requires n = 40 for an effect-size of 0.32 at a statistical power of 0.80.

The study is organized in two phases interspersed by an interim data analysis and report. In phase 1, twenty-four subjects are enrolled in the study (n = 12 per experimental group). All subjects participate in the full study protocol, including the needle biopsies of m. vastus lateralis. At the end of phase 1 an intermediate data analysis is performed on all the primary and secondary endpoints. In case the output of this intermediate data analysis in completely 'negative', the study is terminated at n = 24. In case of relevant positive results from some or more primary or secondary endpoints, a second phase is started with n = 16 in order to increase the power of the study (n = 20 per group).

### 3. Outcome

### 3.1. Study endpoints

### 3.1.1. Primary endpoints

Change in countermovement jump performance over the different measurement points:
- between the pretest and the posttest
- between the pretest and TR1+48h
- between TR1+48h and the posttest (TR17+48h)

### 3.1.2. Secondary endpoints

- Local and systemic immune cell and inflammation markers
- Change in maximal isometric knee-extension force between the different time-points in the right and left leg
- Change in pain perception (VAS) at muscle level between the different time-points in the right and left leg during drop jumps
- Change in pain perception (palpation, VAS and VISA-Q) between the different timepoints at the level of the right and left patellar tendon Change in anteroposterior tendon thickness (millimeters) between the different time-points in the right and left patellar tendon.
- Change in modified Ohberg score between the different time-points in the right and left patellar tendon.
- Change in muscle integrin, desmin, and dystrophin content between the different timepoints in muscle biopsies obtained from the right and left m. vastus lateralis
- Change in blood markers of muscle damage, muscle collagen turnover, muscle extracellular matrix cleavage, and inflammation between the different time-points.

### 4. Results

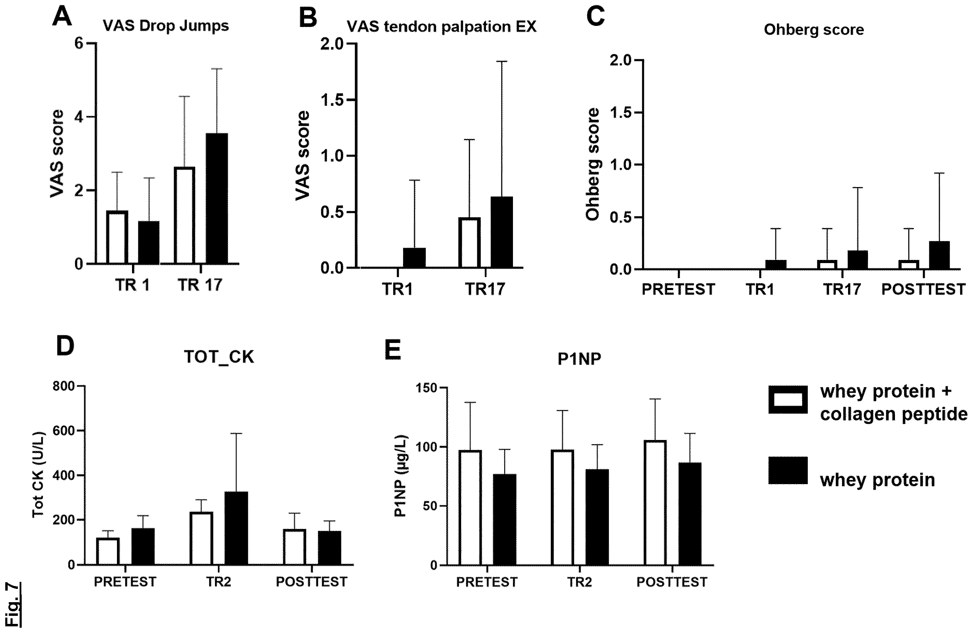

As shown in FIG. 7A the muscle pain perception at training session (TR) 17 was reduced in subjects taking the combination of whey protein/collagen peptide as compared to subjects taking whey protein alone.

As shown in Fig. 7B, the patellar tendon pain perception following palpation at TR1 and TR17 was reduced in subjects taking the combination of whey protein/collagen peptide as compared to subjects taking whey protein alone.

As shown in Fig. 7C, the patellar tendon thickness and neovascularization (Ohberg score) at TR1, TR17, and posttest was reduced in subjects taking the combination of whey protein/collagen peptide as compared to subjects taking whey protein alone. This indicates reduced tendon inflammation and damage for the combination of whey protein/collagen peptide.

As shown in Fig. 7D, the amount of total creatine kinase (CK) in the blood at TR2 was reduced in subjects taking the combination of whey protein/collagen peptide as compared to subjects taking whey protein alone. This indicates reduced muscle damage for the combination of whey protein/collagen peptide.

As shown in Fig. 7E, the amount of procollagen type-1 polypeptidase (P1NP) in the blood at pretest, TR2 and posttest was increased in subjects taking the combination of whey protein/collagen peptide as compared to subjects taking whey protein alone. This indicates that there is an improved collagen repair for the combination of whey protein/collagen peptide.

### 5. Conclusion

This clinical study further establishes the synergistic effects of collagen peptide and whey protein on downplaying tissue inflammation and damage in athletes undergoing strenuous exercise (at least in muscle and tendon), by showing
- an alleviation in inflammation;
- an improvement in the functional tissue capacity and integrity;
- an improvement in the collagen repair;
- a reduction in pain; and
- a reduction in signs of functional tissue damage.

## Claims

1. Combination comprising collagen peptide and whey protein for use in decreasing inflammation in skeletal muscle inflammation.

2. Combination for use according to claim 1, wherein the skeletal muscle inflammation is induced by exercise.

3. Combination for use according to claim 2, wherein the exercise is exercise overload, strength training, and/or sports.

4. Combination for use according to any one of the previous claims, wherein the skeletal muscle inflammation is in one or more conditions selected from the group consisting of exerciseinduced muscle damage, delayed onset muscle soreness, and inflammatory myopathy.

5. Combination for use according to any one of the previous claims, wherein the skeletal muscle inflammation is **characterised by** one or more symptoms selected from the group consisting of:
- reduced skeletal muscle tissue function;
- reduced skeletal muscle tissue repair;
- skeletal muscle tissue loss;
- skeletal muscle tissue soreness; and/or
- skeletal muscle tissue pain.

6. Combination for use according to any one of the preceding claims, wherein:
- the collagen peptide is derived from collagen obtained from animal skin, cartilage, bone and/or connective tissue; and/or
- the collagen peptide is derived from porcine, bovine and/or fish collagen.

7. Combination for use according to any one of the preceding claims, wherein the collagen peptide has a mean molecular weight between 1500 Da and 6000 Da, preferably a mean molecular weight between 1500 Da and 3000 Da or a mean molecular weight between 3000 Da and 6000 Da.

8. Combination for use according to any one of the preceding claims, wherein:
- the whey protein is acid whey or sweet whey; and/or
- the whey protein is provided as a whey protein isolate or a whey protein concentrate.

9. Combination for use according to any one of the preceding claims, wherein the collagen peptide is present in the combination in an amount of between 0.5 g and 200 g, preferably between 1 g and 100 g, more preferably between 2 g and 50 g, most preferably between 5 g and 25 g, and the whey protein is present in the combination in an amount of between 0.1 g and 200 g, preferably between 1 g and 100 g, more preferably between 5 g and 75 g, most preferably between 15 g and 50 g, wherein the amount is the dry weight amount per unit dose.

10. Combination for use according to any one of the preceding claims, wherein the collagen peptide is present in the combination in a daily amount of between 0.5 g and 200 g, preferably between 1 g and 100 g, more preferably between 2 g and 50 g, most preferably between 5 g and 25 g, and that the whey protein is present in the combination in a daily amount of between 0.1 g and 200 g, preferably between 1 g and 100 g, more preferably between 5 g and 75 g, most preferably between 15 g and 50 g, wherein the daily amount is the total dry weight amount administered to a subject per day.

11. Combination for use according to any one of the previous claims, wherein the combination is provided in a single dosage form or in two or more separate dosage forms.

12. Combination for use
according to claim 11, wherein the two or more separate dosage forms contain independently collagen peptide, independently whey protein, or a combination of collagen peptide and whey protein.

13. Combination for use according to any one of claims 10-12, wherein the daily amount of collagen peptide and the daily amount of whey protein are administered within a 24 hours timeframe, preferably within a 12 hours timeframe, most preferably within a 6 hours timeframe.

14. Combination for use according to any one of the preceding claims, wherein the collagen peptide and the whey protein are provided in a food formulation, feed formulation, food supplement formulation, feed supplement formulation, or pharmaceutical formulation, preferably a food supplement formulation.

15. Combination for use according to any one of the preceding claims, wherein the collagen peptide and the whey protein are provided in a solid dosage form such as a capsule, a tablet, or a powder, preferably a powder.

## Patentansprüche

1. Kombination, umfassend Kollagenpeptid und Molkeprotein, zur Verwendung bei der Verringerung von Entzündungen in der Skelettmuskulatur.

2. Kombination zur Verwendung nach Anspruch 1, wobei die Entzündung der Skelettmuskulatur durch körperliche Betätigung ausgelöst wird.

3. Kombination zur Verwendung nach Anspruch 2, wobei es sich bei der körperlichen Betätigung um Überlastung, Krafttraining und/oder Sport handelt.

4. Kombination zur Verwendung nach einem der vorstehenden Ansprüche, wobei die Entzündung der Skelettmuskulatur bei einem oder mehreren Zuständen auftritt, ausgewählt aus der Gruppe bestehend aus trainingsbedingten Muskelschäden, verzögert einsetzendem Muskelkater und entzündlicher Myopathie.

5. Kombination zur Verwendung nach einem der vorstehenden Ansprüche, wobei die Entzündung der Skelettmuskulatur durch ein oder mehrere Symptome gekennzeichnet ist, ausgewählt aus der Gruppe bestehend aus:
- eingeschränkter Funktion des Skelettmuskelgewebes;
- eingeschränkter Reparatur des Skelettmuskelgewebes;
- Verlust von Skelettmuskelgewebe;
- Muskelkater der Skelettmuskulatur und/oder
- Schmerzen im Skelettmuskelgewebe.

6. Kombination zur Verwendung nach einem der vorhergehenden Ansprüche, wobei:
- das Kollagenpeptid aus Kollagen gewonnen wird, das aus tierischer Haut, Knorpel, Knochen und/oder Bindegewebe stammt, und/oder
- das Kollagenpeptid aus Schweine-, Rinder- und/oder Fischkollagen gewonnen wird.

7. Kombination zur Verwendung nach einem der vorhergehenden Ansprüche, wobei das Kollagenpeptid ein mittleres Molekulargewicht zwischen 1500 Da und 6000 Da, vorzugsweise ein mittleres Molekulargewicht zwischen 1500 Da und 3000 Da oder ein mittleres Molekulargewicht zwischen 3000 Da und 6000 Da aufweist.

8. Kombination zur Verwendung nach einem der vorhergehenden Ansprüche, wobei:
- es sich bei dem Molkenprotein um Sauermolke oder Süßmolke handelt, und/oder
- das Molkenprotein als Molkenproteinisolat oder als Molkenproteinkonzentrat bereitgestellt wird.

9. Kombination zur Verwendung nach einem der vorhergehenden Ansprüche, wobei das Kollagenpeptid in der Kombination in einer Menge zwischen 0,5 g und 200 g, vorzugsweise zwischen 1 g und 100 g, mehr bevorzugt zwischen 2 g und 50 g, am meisten bevorzugt zwischen 5 g und 25 g, und das Molkenprotein in der Kombination in einer Menge zwischen 0,1 g und 200 g, vorzugsweise zwischen 1 g und 100 g, mehr bevorzugt zwischen 5 g und 75 g, am meisten bevorzugt zwischen 15 g und 50 g, vorliegt, wobei es sich bei der Menge um die Trockengewichtsmenge pro Dosiseinheit handelt.

10. Kombination zur Verwendung nach einem der vorhergehenden Ansprüche, wobei das Kollagenpeptid in der Kombination in einer täglichen Menge zwischen 0,5 g und 200 g, vorzugsweise zwischen 1 g und 100 g, mehr bevorzugt zwischen 2 g und 50 g, am meisten bevorzugt zwischen 5 g und 25 g, vorliegt, und wobei das Molkenprotein in der Kombination in einer täglichen Menge zwischen 0,1 g und 200 g, vorzugsweise zwischen 1 g und 100 g, mehr bevorzugt zwischen 5 g und 75 g, am meisten bevorzugt zwischen 15 g und 50 g, vorliegt, wobei es sich bei der täglichen Menge um die gesamte Trockengewichtsmenge handelt, die einem Individuum pro Tag verabreicht wird.

11. Kombination zur Verwendung nach einem der vorstehenden Ansprüche, wobei die Kombination in einer einzigen Darreichungsform oder in zwei oder mehreren getrennten Darreichungsformen bereitgestellt wird.

12. Kombination zur Verwendung nach Anspruch 11, wobei die zwei oder mehreren getrennten Darreichungsformen unabhängig voneinander Kollagenpeptid, unabhängig voneinander Molkenprotein oder eine Kombination aus Kollagenpeptid und Molkenprotein enthalten.

13. Kombination zur Verwendung nach einem der Ansprüche 10 bis 12, wobei die Tagesmenge an Kollagenpeptid und die Tagesmenge an Molkenprotein innerhalb eines Zeitrahmens von 24 Stunden, vorzugsweise innerhalb eines Zeitrahmens von 12 Stunden, am meisten bevorzugt innerhalb eines Zeitrahmens von 6 Stunden, verabreicht werden.

14. Kombination zur Verwendung nach einem der vorhergehenden Ansprüche, wobei das Kollagenpeptid und das Molkenprotein in einer Lebensmittelformulierung, Futtermittelformulierung, Nahrungsergänzungsformulierung, Futterergänzungsformulierung oder pharmazeutischen Formulierung, vorzugsweise einer Nahrungsergänzungsformulierung, bereitgestellt werden.

15. Kombination zur Verwendung nach einem der vorhergehenden Ansprüche, wobei das Kollagenpeptid und das Molkenprotein in einer festen Darreichungsform wie etwa einer Kapsel, einer Tablette oder einem Pulver, vorzugsweise einem Pulver, bereitgestellt werden.

## Revendications

1. Combinaison comprenant un peptide de collagène et une protéine de lactosérum, pour une utilisation pour diminuer l'inflammation dans l'inflammation du muscle squelettique.

2. Combinaison pour une utilisation selon la revendication 1, dans laquelle l'inflammation du muscle squelettique est induite par l'exercice.

3. Combinaison pour une utilisation selon la revendication 2, dans laquelle l'exercice est une surcharge d'exercice, un entraînement musculaire et/ou un sport.

4. Combinaison pour une utilisation selon l'une quelconque des revendications précédentes, dans laquelle l'inflammation du muscle squelettique est dans un ou plusieurs états choisis dans le groupe constitué par une lésion musculaire induite par l'exercice, une douleur musculaire à apparition retardée et une myopathie inflammatoire.

5. Combinaison pour une utilisation selon l'une quelconque des revendications précédentes, dans laquelle l'inflammation du muscle squelettique est **caractérisée par** un ou plusieurs symptômes choisis dans le groupe constitué de :
- réduction de la fonction du tissu musculaire squelettique ;
- réduction de la réparation du tissu musculaire squelettique ;
- perte de tissu musculaire squelettique ;
- douleur du tissu musculaire squelettique ; et/ou
- douleur du tissu musculaire squelettique.

6. Combinaison pour une utilisation selon l'une quelconque des revendications précédentes, dans laquelle :
- le peptide de collagène est dérivé de collagène obtenu à partir de peau, de cartilage, d'os et/ou de tissu conjonctif d'animaux ; et/ou
- le peptide de collagène est dérivé de collagène porcin, bovin et/ou de poisson.

7. Combinaison pour une utilisation selon l'une quelconque des revendications précédentes, dans laquelle le peptide de collagène a un poids moléculaire moyen compris entre 1 500 Da et 6 000 Da, de préférence un poids moléculaire moyen compris entre 1 500 Da et 3 000 Da ou un poids moléculaire moyen compris entre 3 000 Da et 6 000 Da.

8. Combinaison pour une utilisation selon l'une quelconque des revendications précédentes, dans laquelle :
- la protéine de lactosérum est du lactosérum acide ou du lactosérum doux ; et/ou
- la protéine de lactosérum est fournie sous forme d'isolat de protéine de lactosérum ou de concentré de protéine de lactosérum.

9. Combinaison pour une utilisation selon l'une quelconque des revendications précédentes, dans laquelle le peptide de collagène est présent dans la combinaison en une quantité comprise entre 0,5 g et 200 g, de préférence entre 1 g et 100 g, plus préférablement entre 2 g et 50 g, le plus préférablement entre 5 g et 25 g, et la protéine de lactosérum est présente dans la combinaison en une quantité comprise entre 0,1 g et 200 g, de préférence entre 1 g et 100 g, plus préférablement entre 5 g et 75 g, le plus préférablement entre 15 g et 50 g, la quantité étant la quantité en poids sec par dose unitaire.

10. Combinaison pour une utilisation selon l'une quelconque des revendications précédentes, dans laquelle le peptide de collagène est présent dans la combinaison en une quantité quotidienne comprise entre 0,5 g et 200 g, de préférence entre 1 g et 100 g, plus préférablement entre 2 g et 50 g, le plus préférablement entre 5 g et 25 g, et la protéine de lactosérum est présente dans la combinaison en une quantité quotidienne comprise entre 0,1 g et 200 g, de préférence entre 1 g et 100 g, plus préférablement entre 5 g et 75 g, le plus préférablement entre 15 g et 50 g, la quantité quotidienne étant la quantité totale en poids sec administrée à un sujet par jour.

11. Combinaison pour une utilisation selon l'une quelconque des revendications précédentes, dans laquelle la combinaison est fournie sous une forme posologique unique ou sous deux ou plusieurs formes posologiques distinctes.

12. Combinaison pour une utilisation selon la revendication 11, dans laquelle les deux formes posologiques distinctes ou plus contiennent indépendamment un peptide de collagène, indépendamment une protéine de lactosérum ou une combinaison de peptide de collagène et de protéine de lactosérum.

13. Combinaison pour une utilisation selon l'une quelconque des revendications 10 à 12, dans laquelle la quantité quotidienne de peptide de collagène et la quantité quotidienne de protéine de lactosérum sont administrées dans un laps de temps de 24 heures, de préférence dans un laps de temps de 12 heures, le plus préférablement dans un laps de temps de 6 heures.

14. Combinaison pour une utilisation selon l'une quelconque des revendications précédentes, dans laquelle le peptide de collagène et la protéine de lactosérum sont fournis dans une formulation alimentaire, une formulation alimentaire pour animaux, une formulation de supplément alimentaire, une formulation de supplément alimentaire pour animaux ou une formulation pharmaceutique, de préférence, une formulation de supplément alimentaire.

15. Combinaison pour une utilisation selon l'une quelconque des revendications précédentes, dans laquelle le peptide de collagène et la protéine de lactosérum sont fournis sous une forme posologique solide telle qu'une capsule, un comprimé ou une poudre, de préférence une poudre.
